(19) Europäisches Patentamt
European Patent Office
Office européen des brevets

(11) **EP 4 451 279 A1**

(12) **EUROPEAN PATENT APPLICATION**

(43) Date of publication:
**23.10.2024   Bulletin 2024/43**

(21) Application number: **23169108.0**

(22) Date of filing: **21.04.2023**

(51) International Patent Classification (IPC):
***G16C 20/30*** (2019.01)

(52) Cooperative Patent Classification (CPC):
**G16C 20/30;** C09K 8/584; C11D 1/66; C11D 11/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **BASF SE**
**67056 Ludwigshafen am Rhein (DE)**

(72) Inventors:
• **Dailey, James S**
  **Wyandotte, Michigan 48192 (US)**
• **Jamieson, Mitchell**
  **Wyandotte, Michigan 48192 (US)**
• **Wang, Shefang**
  **Ford Lee, 07024 (US)**

(74) Representative: **Maiwald GmbH**
**Elisenhof**
**Elisenstraße 3**
**80335 München (DE)**

(54) **USE OF NONLINEAR MIXING CALCULATIONS FOR PREDICTION OF HYDROPHILIC LIPOPHILIC DIFFERENCE VALUES IN DIGITAL APPLICATIONS**

(57)     The following relates generally to (i) determining characteristic values of nonionic surfactant mixes, and/or (ii) determining an optimal surfactant. In some embodiments, one or more processors receive indications of first and second nonionic surfactants to be used to form a surfactant mix. The one or more processors may then determine a two-parameter or three-parameter nonlinear equation to calculate a characteristic value of the surfactant mix. In some examples, the nonlinear equation includes a variable of a molar fraction of either the first nonionic surfactant or the second nonionic surfactant.

## Description

BACKGROUND

The Hydrophilic Lipophilic Difference (HLD) equation creates a mathematical representation of the microstructure of the self-assembly for a system containing surfactant, oil, and water. This mathematical representation can be used for a wide range of applications including emulsion polymerization, laundry, hard surface cleaning, enhanced oil recovery, and more. For example, this mathematical representation is useful in determining solutions to clean particular kinds of oils from particular kinds of surfaces.

However, the HLD equation relies on knowledge of a characteristic value of a surfactant mix, sometimes referred to as the characteristic curvature; and current systems cannot infer accurate characteristic values for surfactant mixes, in particular for nonionic surfactant mixes.

The systems and methods disclosed herein provide solutions to these problems and others.

SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

In one aspect, a computer-implemented method for determining a characteristic value of a nonionic surfactant mix may be provided. The method may include: receiving, via one or more processors: (i) an indication of a first nonionic surfactant, and (ii) a first molar fraction of the first nonionic surfactant; receiving, via the one or more processors: (i) an indication of a second nonionic surfactant, and (ii) a second molar fraction of the second nonionic surfactant; and determining, via the one or more processors, a characteristic value of a surfactant mix including the first molar fraction of the first nonionic surfactant, and the second molar fraction of the second nonionic surfactant, and wherein the characteristic value is determined by inputting, into a nonlinear equation, either the first molar fraction of the first nonionic surfactant or the second molar fraction of the second nonionic surfactant.

In another aspect, a computer-implemented method for determining an optimal surfactant may be provided. The method may include: receiving, via one or more processors: (i) an indication of a base nonionic surfactant, and (ii) a molar fraction of the base nonionic surfactant; receiving, via the one or more processors, a desired hydrophilic lipophilic difference (HLD) range of a solution including a desired surfactant mix comprising the base nonionic surfactant; retrieving, via the one or more processors, a list of surfactants, from a surfactant database; and determining, via the one or more processors, at least one recommended surfactant by: creating nonlinear equations, wherein the nonlinear equations: (i) correspond, respectively, to nonionic surfactants in the list of surfactants, and (ii) are based on (a) the base nonionic surfactant and (b) the molar fraction of the base nonionic surfactant; and determining at least one recommended surfactant based on: (i) the desired HLD range, and (ii) the created nonlinear equations.

In yet another aspect, a computer system for determining a characteristic value of a nonionic surfactant mix may be provided. The computer system may include one or more processors configured to: receive: (i) an indication of a first nonionic surfactant, and (ii) a first molar fraction of the first nonionic surfactant; receive: (i) an indication of a second nonionic surfactant, and (ii) a second molar fraction of the second nonionic surfactant; and determine, a characteristic value of a surfactant mix including the first molar fraction of the first nonionic surfactant, and the second molar fraction of the second nonionic surfactant, and wherein the characteristic value is determined by inputting, into a nonlinear equation, either the first molar fraction of the first nonionic surfactant or the second molar fraction of the second nonionic surfactant.

BRIEF DESCRIPTION OF THE DRAWINGS

Figure 1 illustrates example phase behavior results, which may be used to determine a scaling constant (k), and a characteristic value $\sigma_{mix}$.

Figure 2 illustrates example $\sigma_{mix}$ predictions for mixtures of Lutensol TO 3 and Lutensol TO 7 using linear mixing, with experimentally determined result included.

Figure 3 illustrates example linear and nonlinear predictions for $\sigma_{mix}$, as well as an experimentally determined result.

Figure 4 illustrates experimental $\sigma_{mix}$ values for mixtures of Lutensol TO 3 and Lutensol TO 7, with linear mixing predictions included.

Figure 5 illustrates experimental $\sigma_{mix}$ values for mixtures of Lutensol TO 3 and Lutensol TO 7, along with the two nonlinear mixing equations, with linear mixing predictions included.

Figure 6 illustrates example predicted (Exp2P) and experimental $\sigma_{mix}$ values for mixtures of Lutensol TO 3 and Lutensol A 65 N, with linear mixing predictions included.

Figure 7 illustrates experimental $\sigma_{mix}$ values for mixtures of Lutensol TO 3 and Lutensol A 65 N, along with the two nonlinear mixing equations, with linear mixing predictions included.

Figure 8 illustrates experimental S* values for mixtures of Lutensol TO 7 and Lutensol TO 12, along with the two nonlinear mixing equations, with linear mixing predictions included.

Figure 9 illustrates predicted (Exp2P) $\sigma_{mix}$ values for mixtures of Lutensol TO 3 and Lutensol AO 3, with linear mixing predictions included.

Figure 10 illustrates regressor-dependent predicted (Exp2P) $\sigma_{mix}$ values for mixtures of Lutensol TO 3 and Lutensol AO 3, with linear mixing predictions included.

Figure 11 illustrates regressor-dependent predicted (Exp2P) and experimental $\sigma_{mix}$ values for mixtures of Lutensol TO 3 and Lutensol AO 3, with linear mixing predictions included.

Figure 12 illustrates an example system for determining characteristic value of a nonionic surfactant mix and/or determining an optimal surfactant.

Figure 13 illustrates an example screen for determining a characteristic value of a surfactant mix including two surfactants.

Figure 14 illustrates an example screen for determining a characteristic value of a surfactant mix including three surfactants.

Figure 15 illustrates an example screen for determining an optimal surfactant.

Figure 16 illustrates an example screen for determining an optimal surfactant mix including two surfactants.

Figure 17 illustrates an example method for determining a surfactant mix including two surfactants.

Figure 18 illustrates an example screen displaying an optimum temperature.

Figure 19 illustrates an example method for determining a surfactant mix including three surfactants.

Figure 20 illustrates an example method including determining whether to use linear equation(s) or nonlinear equation(s).

Figure 21 illustrates an example method for determining recommended surfactants, including receiving a desired HLD range.

Figure 22 illustrates an example screen for determining a surfactant.

Figure 23 illustrates an example screen for determining a surfactant for a surfactant mix comprising two surfactants.

Advantages will become more apparent to those skilled in the art from the following description of the preferred embodiments which have been shown and described by way of illustration. As will be realized, the present embodiments may be capable of other and different embodiments, and their details are capable of modification in various respects. Accordingly, the drawings and description are to be regarded as illustrative in nature and not as restrictive.

DETAILED DESCRIPTION

The present embodiments relate to, *inter alia,* (i) determining characteristic values of nonionic surfactant mixes, and (ii) determining an optimal surfactant.

By way of broad overview, the Hydrophilic Lipophilic Difference (HLD) equation creates a mathematical representation of the microstructure of the self-assembly for a system containing surfactant, oil, and water. The HLD is a dimensionless number, where negative values predict the presence of oil-swollen micelles dispersed in a continuous aqueous phase (oil-in-water; $\mu$E, Winsor Type I) and positive values predict the presence of water-swollen reverse micelles dispersed in a continuous oil phase (emulsion of water-in-oil; $\mu$E, Winsor Type II). A value at or near zero indicates the system is at the phase inversion point, where a bicontinuous network of oil and water channels forms (Winsor Type III/IV). Put simply, the HLD equation can robustly predict the behavior of a complex system by considering several variables, including the salinity of the aqueous phase, oiliness of the oil (e.g., the EACN of the oil), temperature, and surfactant hydrophilicity/hydrophobicity. More particularly, the HLD equation is:

$$\mathrm{HLD} = f(S) - \mathrm{k}(\mathrm{EACN}) + c_T(\mathrm{T} - 25) + \sigma \quad (1)$$

Where HLD is the hydrophilic lipophilic difference, $f(S)$ is a function of salinity, EACN is the effective alkane carbon number of the oil, T is the temperature (in °C), and $\sigma$ is the characteristic value of the surfactant, also known as the characteristic curvature, Cc. k and $c_T$ are both scaling constants.

$f(S)$ and $c_T$ are dependent on the type of surfactant (i.e., ionic, nonionic, ethoxylate, APG [Alkyl polyglycoside generally, or alkyl polyglucoside if the sugar head group is glucose], etc.). Typical values are given in Table 1 below:

Table 1: $f(S)$ and $c_T$ values for surfactants of various types.

| Surfactant Type | $f(S)$ | $c_T$ |
|---|---|---|
| Ionic | ln(S+SurfSal) | -0.01 |
| Ethoxylate | 0.13×S | 0.06 |
| APG | ~0 | 9 |

These values can be found throughout the literature to remain relatively consistent; however, the values of k and $\sigma$ can vary dramatically depending on the surfactant because they depend on the hydrophilicity of the surfactant head group and the hydrophobicity of the surfactant tail. According to Acosta and Bhakta, [Acosta EJ, Bhakta AS. (2009) The HLDNAC Model for Mixtures of Ionic and Nonionic Surfactants. J Surfactants Deterg, 12, 7-19.], k typically ranges from 0.1 to 0.2, but Withayapanyanon et al. [Witthayapanyanon A, Harwell JH, Sabatini DA. (2008) Hydrophilic-lipophilic deviation (HLD) method for characterizing conventional and extended surfactants. J Colloid Interface Sci, 325, 259-266.] report values as low as 0.062.

The HLD can also be applied to mixtures of surfactants, which is important because in many commercial applications it is necessary to use more than one surfactant to achieve the desired properties; for example, a co-surfactant may be added to raise the cloud point or to reduce foaming. It has been generally believed and reported [Acosta and Bhakta] [Witthayapanyanon A, Harwell JH, Sabatini DA.] [Salager JL, Morgan JC, Schechter RS, Wade WH, Vasquez E. (1979) Optimum Formulation of Surfactant/Water/Oil Systems for Minimum Interfacial Tension or Phase Behavior. Soc Pet Eng J, 19(02), 107-115.] [Salager JL, Bourrel M, Schechter RS, Wade WH. (1979) Mixing Rules for Optimim Phase-Behavior Formulations of Surfactant/Oil/Water Systems. Soc Pet Eng J, 19(05), 271-278.] that the HLD of mixtures of similar surfactants can be calculated using the so-called linear mixing equations (Equations 2-4 below), but to date there has been a lack of information regarding how mixtures of technical grade nonionic surfactants (including ethoxylated nonionic surfactants) behave.

$$\mathrm{HLD}_{\mathrm{mix}} = \sum \chi_i \mathrm{HLD}_i \qquad (2)$$

$$\mathrm{k}_{\mathrm{mix}} = \sum \chi_i \mathrm{k}_i \qquad (3)$$

$$\sigma_{\mathrm{mix}} = \sum \chi_i \sigma_i \qquad (4)$$

Where $\chi$ refers to the molar fraction of the surfactant.

For technical grade ethoxylated surfactants, which are typically polydisperse, the low molecular weight oligomers present, as well as any residual alcohol, lead to a partitioning effect that ultimately results in the $\sigma$ value exhibiting a concentration dependence. As disclosed herein, every ethoxylated surfactant has a $\sigma$ value that can be expressed, in general terms, as

$$\sigma = a\ln(x) + d \qquad (5)$$

Where x is the surfactant concentration (percent weight/volume with respect to oil) and a and d are constants that may be experimentally determined for each surfactant. As surfactant concentration decreases, the effective a value becomes more negative. This presents an issue when mixing ethoxylated surfactants, namely what concentration to use to predict the a of the blend using the linear mixing equation. For a 1:1 mixture of two surfactants where the total surfactant concentration is 10%, should the 5% $\sigma$ values or the 10% $\sigma$ values be used in the calculation? One might expect that using the 5% $\sigma$ values would lead to an overestimation of the blend's hydrophilicity (more negative $\sigma$ than reality), whereas using the 10% $\sigma$ values would underestimate its hydrophilicity (more positive $\sigma$ than reality).

Phase behavior studies can shed light on the true nature of these blends, and, by employing several different systems, the HLD framework may be extended by offering a novel mixing rule for systems containing technical grade ethoxylated nonionic surfactants.

Exemplary Experimental Setup According to an Embodiment

The following section describes an example experiment that was run to gather data.

For phase behavior studies of nonionic surfactants, several vials were made that contain surfactant, brine, and oil. Salinity of the brine was used as the scanning variable, and the vials are kept in an incubator to maintain a stable temperature over the course of the equilibration period. Linear alkanes are used as the oil phase, as they have well-defined EACN values. Stock salt solutions were made in 100 mL volumetric flasks at 5% intervals using NaCl and deionized water, where the salinity is expressed as g NaCl per 100 mL aqueous solution. For each blend discussed herein, the individual surfactants were heated and shaken to homogenize, then combined and stirred before being dosed into the vials.

The salt scan procedure started with dosing the appropriate mass of surfactant into several vials. Crimp cap headspace vials were used because they seal very well, which prevents oil loss from evaporation. After the surfactant mixture, brine was added to the vial, followed by deionized water, if necessary, to achieve the desired salinity. Finally, the oil was added. It should be noted that for all experiments discussed in this disclosure, the water/oil ratio (WOR) is 1.

After the oil was added, the cap was crimped to seal the vial. Once each vial in the scan is sealed, they were vortexed for 30 s. After 5 min of dwell time, the vials were each vortexed for another 30 s, then placed in an incubator. The next day, the vials equilibrated to the appropriate temperature, and they were vortexed for 10 s and placed back in the incubator. When several hours passed, the vials were quickly and simultaneously inverted five times and returned to the incubator, where they were left to equilibrate for at least 2 weeks.

The phase boundaries were monitored, and once it was determined that they were stable, the boundaries were marked with a permanent marker. A photo is taken, and then the phase volumes were measured using the ImageJ analysis software. The phase volumes are entered into a spreadsheet template that was developed in accordance with the techniques described herein. The template uses the phase volumes to calculate the salinity at which the microemulsion contains equal volumes of oil and water. This is defined as the optimum salinity, or S*.

At HLD = 0, Equation 1 can be rearranged to calculate the other parameters:

$$f(S^*) + c_T(T - 25) = k(\text{EACN}) - \sigma \qquad (6)$$

If the optimum salinity of a surfactant system is found for several oils, this linear equation can be plotted such that the slope is k and the y-intercept is -σ. If k is known, then $\sigma$ can be calculated directly.

Experimental Results

The K Value

In the first set of experiments, a mixture of Lutensol TO 3 and Lutensol TO 7 was employed to determine the value of k for a blend of nonionic ethoxylated surfactants dosed at 10%. These surfactants were chosen because their individual HLD parameters (k and $\sigma$) were already carefully determined (Table 2). An equal mixture by weight of Lutensol TO 3 and Lutensol TO 7 was used, corresponding to a molar ratio of 0.60 for Lutensol TO 3 and 0.40 for Lutensol TO 7. Using the linear mixing equation (Equation 3), the predicted value of k for this mixture is 0.101 (Equation 7). Using the linear mixing equation (Equation 4), the predicted $\sigma_{mix}$ using their individual 10% $\sigma$ values is -0.21, and the predicted $\sigma_{mix}$ using their 5% $\sigma$ values is -1.63 (Equations 8-9).

$$k_{mix} = (0.60 \times 0.059) + (0.40 \times 0.163) \quad (7)$$

$$= 0.101$$

$$\sigma_{mix} = (0.60 \times 0.74) + (0.40 \times -1.64) \quad (8)$$

$$= -0.21$$

$$\sigma_{mix}(0.60 \times -1.12) + (0.40 \times -2.40) \quad (9)$$

$$= -1.63$$

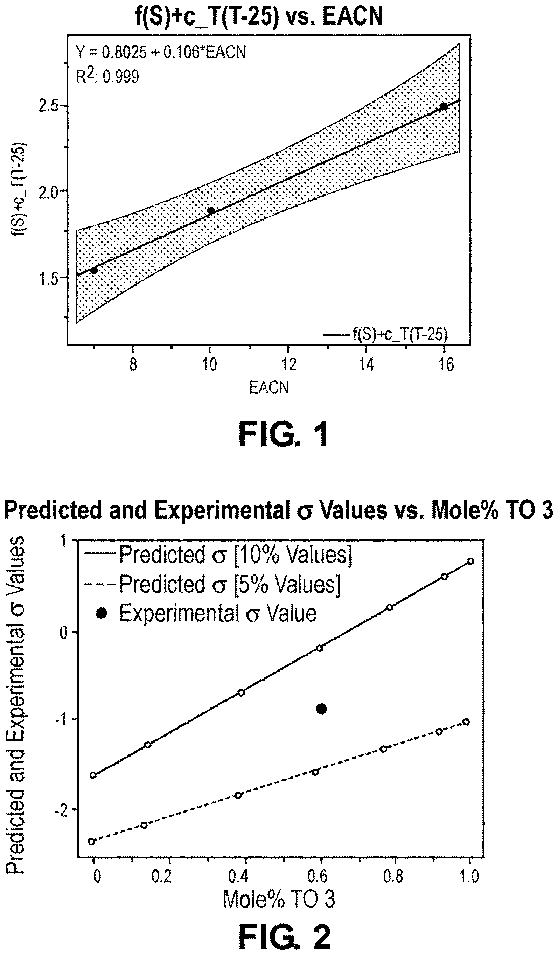

Results for phase behavior scans with three different oils are plotted in Figure 1. The determined $k_{mix}$ (the slope of the line) is found to be 0.106, and the $\sigma_{mix}$ (the negative intercept) is -0.80. Interestingly, the $k_{mix}$ is in line with the prediction from the linear mixing equation. $\sigma_{mix}$, however, is somewhere between the two predicted values calculated using the linear mixing equation. The results showed that when using the 5% $\sigma$ values in the calculation, the prediction was too negative; when using the 10% $\sigma$ values, the predicted $\sigma_{mix}$ was not negative enough.

Table 2: Relevant parameters for the Lutensol TO surfactants used.

|  | Lutensol TO 3 | Lutensol TO 7 |
| --- | --- | --- |
| Molecular Weight [g/mol] | 340 | 510 |
| $\sigma$ [5%] | -1.12 | -2.40 |
| $\sigma$ [10%] | 0.74 | -1.64 |
| $\sigma$ [eqn] | 2.70 * ln(x) - 5.47 | 1.13 * ln(x) - 4.85 |
| k | 0.059 | 0.163 |

The $\sigma_{mix}$ Value

Figure 2 shows a plot of the linear mixing equations that result from predictions of $\sigma_{mix}$ using the 5% and 10% $\sigma$ values for Lutensol TO 3 and Lutensol TO 7. The point between the two lines is the experimentally determined $\sigma_{mix}$ value for that mixture (from Figure 1).

To better understand how $\sigma$ values of mixtures of nonionic surfactants (including ethoxylated surfactants) deviate from linearity, one must look at the results of phase behavior scans for several different mixtures of these two surfactants. Since the total surfactant concentration will be held constant at 10%, the predicted $\sigma_{mix}$ using the 5% values may be disregarded because one knows that for $\chi_{(T03)} = 0$ and $\chi_{(T03)} = 1$ the correct $\sigma_{mix}$ will lie on the predicted $\sigma$ [10% values] line in Figure 2.

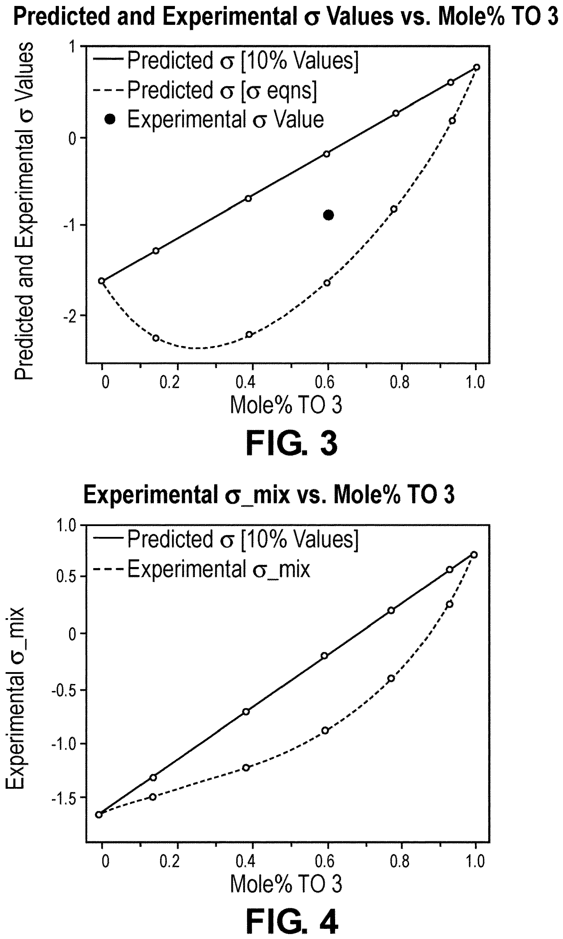

With variations in the concentration of each surfactant in the mixtures to be explored, the following discussion will now consider predicted $\sigma_{mix}$ values that use the concentration-dependent $\sigma$ equations of each surfactant (Figure 3, which uses Equation 5). The calculation for these $\sigma_{mix}$ values applies linear mixing to calculate $k_{mix}$, as this was determined to be an accurate prediction equation in the previous section. This $\sigma_{mix}$ prediction equation is nonlinear since the concentration dependence of $\sigma$ is also nonlinear. However, it still predicts a range of $\sigma_{mix}$ values that are too negative.

The following discussion will examine the observed behavior of several different mixtures of Lutensol TO 3 and Lutensol TO 7, then fit a line, generalize the equation, and apply it to other mixtures to confirm that it applies to other types of nonionic surfactant mixtures.

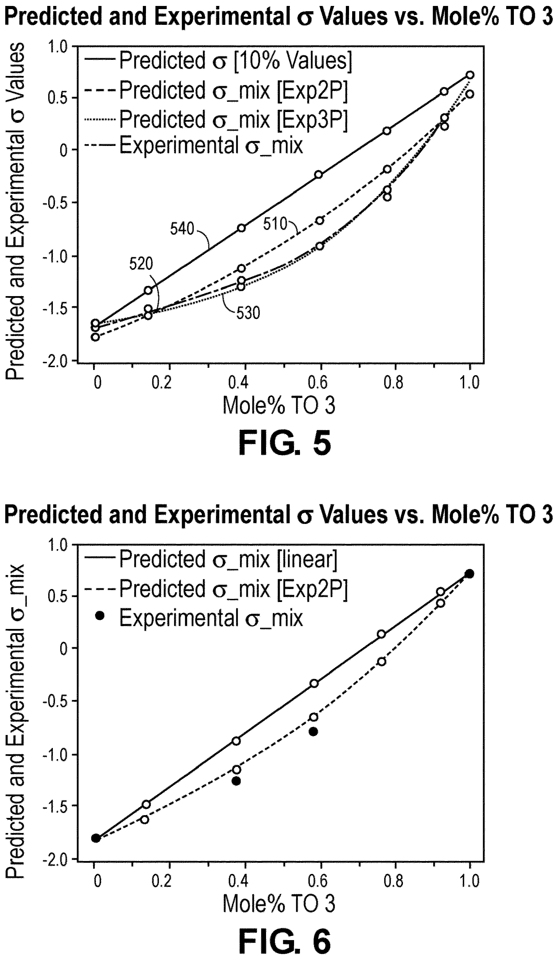

Four more mixtures across the range of $\chi_{TO3}$ were prepared for examination. The results are shown in Figure 4. These experiments thus show that the $\sigma_{mix}$ of these technical grade alcohol ethoxylate blends do not obey the linear mixing rule, instead displaying significant nonlinear character. Embodiments disclosed herein fit two types of nonlinear equations to these results: a two-parameter exponential equation (Exp2P) and a three-parameter exponential equation (Exp3P). These fitted equations are shown in Figure 5.

The two-parameter exponential equation can be generalized as

$$\sigma_{mix} = q + re^{\chi_1} \qquad (10)$$

The three-parameter exponential equation can be generalized as

$$\sigma_{mix} = q + re^{s\chi_1} \qquad (11)$$

Where q is referred to as the "asymptote," r is referred to as the "scale," and s is referred to as the "growth rate." In some embodiments, the Exp2P equation can be defined if the two individual surfactant $\sigma$ values are known, whereas fitting a Exp3P equation requires at least one experimental value of $\sigma_{mix}$. There are pros and cons to each of these nonlinear mixing equations. The Exp2P fit does not require any experiments to use. For example, the starting and ending points of the curve are known because these points correspond to 100% of each individual surfactant; and thus, the system of equations may be solved for q and r. Furthermore, though Exp2P is better than the linear mixing prediction, it is not the best fit ($R^2 = 0.973$) (e.g., it is not as good as Exp3). The Exp3P fit is nearly perfectly predictive ($R^2 = 0.998$), but it may require at least one experiment to use (e.g., the experiment provides the third data point so that the system of equations may be solved for all of q, r, and s).

In the Exp2P equation, q and r can be defined in terms of the individual $\sigma$ values through a process of substitution. The derivation will not be outlined here, but the Exp2P $\sigma_{mix}$ equation for mixtures of polydisperse alcohol ethoxylates, in terms of the individual $\sigma$ values of the components, is

$$\sigma_{mix} = \frac{\sigma_1 - \sigma_2 e}{1-e} + \left(\sigma_2 - \frac{\sigma_1 - \sigma_2 e}{1-e}\right)e^{\chi_1} \qquad (12)$$

Where the individual $\sigma$ values are taken at the concentration of the desired end mixture, e.g., if the desired mixture concentration is 10%, the individual $\sigma$ values in the equation should be the surfactant $\sigma$ values at 10%.

It is a significant achievement to be able to accurately predict and explain the behavior of mixtures of technical grade ethoxylated nonionic surfactants. But the preceding discussion has focused on one system comprised of two surfactants that share the same hydrophobe. The next section examines the extensibility of these nonlinear equation predictions to other types of mixtures. Furthermore, systems with three surfactants will be discussed in greater detail elsewhere herein.

Verification of the Nonlinear Mixing Equations in Other Systems

Now that the preceding discussion has developed a framework for calculating $\sigma_{mix}$ values for blends of polydisperse nonionic surfactants, the following analysis will use other systems to verify that it holds true for a wide variety of mixture types. First, the following will look at mixtures containing surfactants with different hydrophobes, then mixtures of surfactants with higher EO content (average 7-12 EO units) and mixtures with lower EO content (average 3 EO units).

Lutensol TO 3 contains an iC13-based hydrophobe. This was blended at different ratios with Lutensol A 65 N, which contains a different, linear C12,14-based hydrophobe. Both of their $\sigma$ and k values were previously determined, and thus Equation 12 may be used to predict the $\sigma_{mix}$ of various blends. This prediction is shown, along with the experimental results in Figure 6.

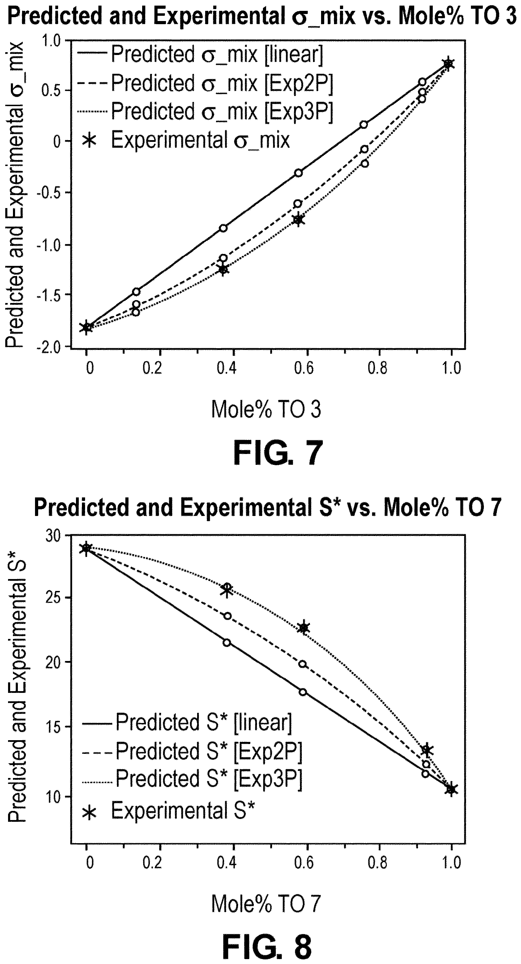

In this case, the Exp2P equation accurately predicted the $\sigma_{mix}$ values of these mixtures ($R^2 = 0.996$). When the Exp3P equation is fitted using only one of the experimental $\sigma_{mix}$ results (Figure 7), the prediction becomes, again, nearly perfect ($R^2 = 1.000$). This $R^2$ would marginally decrease with added data points.

Since the concentration-dependence of the $\sigma$ value is due, in large part, to the lower EO containing oligomers, a blend containing surfactants with higher amounts of EO was used. A blend of Lutensol TO 7 (average 7 units EO) and Lutensol TO 12 (average 12 units EO) was employed to examine whether the blends of higher EO-containing surfactants would display a more linear character. Since the $\sigma$ and k parameters for Lutensol TO 12 have yet to be determined, Figure 8 illustrates a plot like that in Figure 7, using the optimum salinity as the dependent variable instead of $\sigma_{mix}$. The results of these mixtures are just as nonlinear as those mixtures shown previously, where one component of the system only contains 3 EO units.

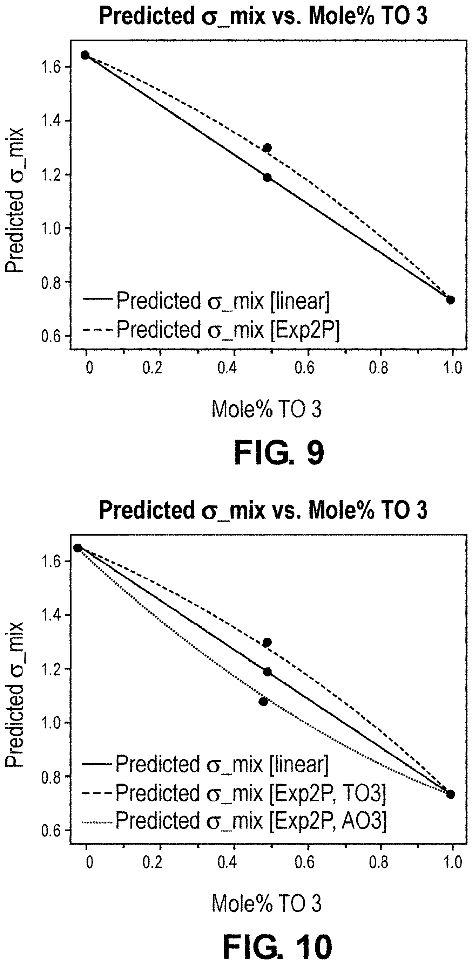

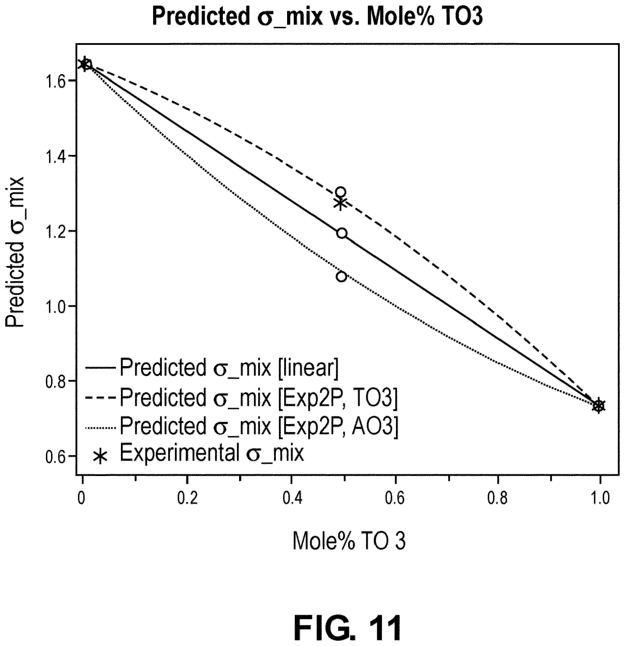

Next, a blend of Lutensol TO 3 (average 3 units EO) and Lutensol AO 3 (average 3 units EO) is examined. The Exp2P predictions are shown in Figure 9. Of note, this is the first blend where the nonlinear equation predicts a more hydrophobic behavior than the linear mixing prediction. In each case before, the blend experimentally behaved more hydrophilic than the linear mixing prediction. However, if the regressor (the mole fraction component used in Equation 12), in this case, was defined as Lutensol AO 3 instead of Lutensol TO 3, the Exp2P predicts that the blend will behave more hydrophilic than linear mixing (Figure 10). The result of this experiment sheds light on how to define the surfactant order in the nonlinear Exp2P equation. The experimental result is shown in Figure 11, and interestingly, it did behave more hydrophobic than linear mixing suggested.

In some embodiments, a caveat is that the surfactant to be defined as the regressor, i.e., surfactant 1 of the blend, should be the surfactant that is more positive, unless blending two surfactants that both have $\sigma > 0$, in which case the surfactant to be defined as the regressor should be the surfactant that is less positive. This is true at least of all the blends tested for purposes of this disclosure. It should be noted that, in some embodiments, this caveat does not apply for the Exp3P because an experiment must already be done to fit an equation, so no assumptions must be made because the character of the experimental blend used is considered in the fitted equation.

Example System For Determining Characteristic Value Of A Nonionic Surfactant Mix And/or Determining an Optimal Surfactant

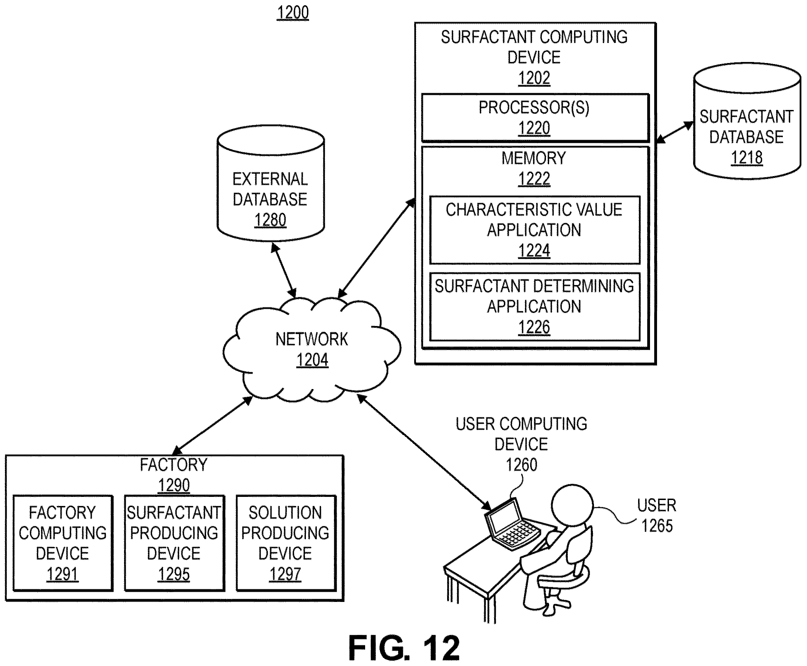

Figure 12 depicts an exemplary computing environment 1200 in which the techniques disclosed herein may be implemented, according to some aspects. The computing environment 1200 may include a surfactant computing device 1202, which, in some aspects, may implement the techniques described herein. For example, the surfactant computing device 1202 may determine characteristic values of nonionic surfactant mixes, and/or determine an optimal surfactant.

The surfactant computing device 1202 may include one or more processors 1220 such as one or more microprocessors, controllers, and/or any other suitable type of processor. The surfactant computing device 1202 may further include a memory 1222 (e.g., volatile memory, non-volatile memory) accessible by the one or more processors 1220, (e.g., via a memory controller). The one or more processors 1220 may interact with the memory 1222 to obtain and execute, for example, computer-readable instructions stored in the memory 1222. Additionally or alternatively, computer-readable instructions may be stored on one or more removable media (e.g., a compact disc, a digital versatile disc, removable flash memory, etc.) that may be coupled to the surfactant computing device 1202 to provide access to the computer-readable instructions stored thereon. In particular, the computer-readable instructions stored on the memory 1222 may include applications, such as characteristic value application 1224, and/or surfactant determining application 1226.

The characteristic value application 1224 may include instructions for, *inter alia,* determining one or more characteristic values $\sigma$ of surfactants. For example, as discussed in further detail elsewhere herein, the characteristic value application 1224 may use the two-parameter equation (Equation 10), or the three-parameter equation (Equation 11) to calculate the characteristic value of a nonionic surfactant mix, $\sigma_{mix}$.

To this end, to calculate the characteristic value, the characteristic value application 1224 may also use information received from a user 1265. The information may be received via the user computing device 1260, which may be any suitable computing device. Examples of the user computing device 1260 include a personal computer, a tablet, a smartphone, a phablet, etc.

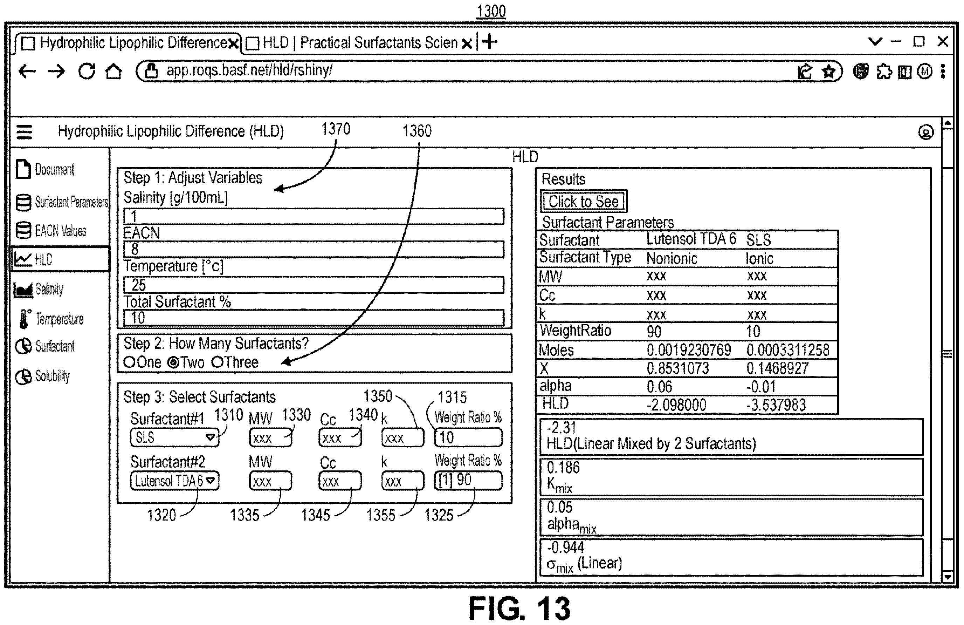

Figure 13 shows an example screen 1300 into which the user 1265 may enter such information. For example, the user 1265 may enter a first surfactant 1310 (which may be ionic or nonionic) and/or a second surfactant 1320 (which also may be ionic or nonionic). The user 1265 may further enter a first molar fraction 1325 of the first surfactant 1320, and/or enter a second molar fraction 1325 of the second surfactant 1320. In some embodiments, upon selection of a surfactant, information may be auto populated. Examples of information that may be auto populated are molar weight 1330, 1335, characteristic value $\sigma$ 1340, 1345 (in some embodiments, this is displayed as "Cc" for characteristic curvature rather than displayed as "$\sigma$"), and equilibrium constant k, 1350, 1355. It may be noted that the user 1265 may enter the surfactant via any suitable technique (e.g., using a dropdown arrow to select from a list, typing the surfactant name into the box, etc.).

Furthermore, the user 1265 may enter additional information into entry box 1370, such as salinity of the solution that the surfactant mix will be part of, EACN of the oil, temperature of the solution (e.g., optimum temperature), total surfactant percentage in the solution, etc.

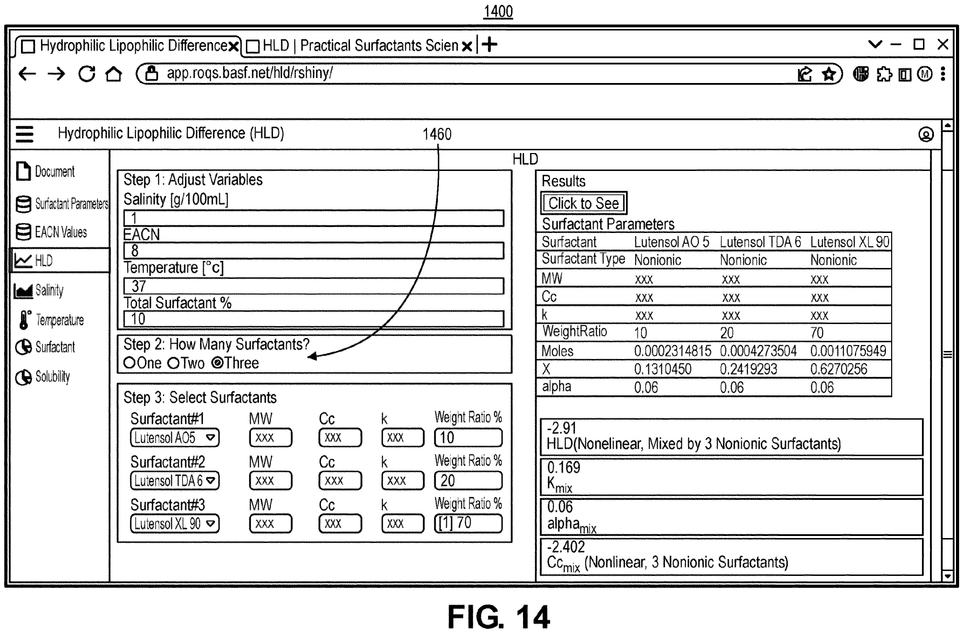

In addition, the user 1265 may specify the number of surfactants, such as by entering the number of surfactants into the entry box 1360. Figure 14 shows an example screen 1400 where the number of surfactants has been entered as three in the entry box 1460.

In addition, the example screen 1400 illustrates an implementation where the notation "Cc" is used rather than "$\sigma$."

Returning now to Figure 12, the surfactant determining application 1226 may include instructions for, *inter alia,* determining an optimal surfactant. To determine the optimal surfactant, the surfactant determining application 1226 may use information received from a user 1265 (e.g., entered via the user computing device 1260).

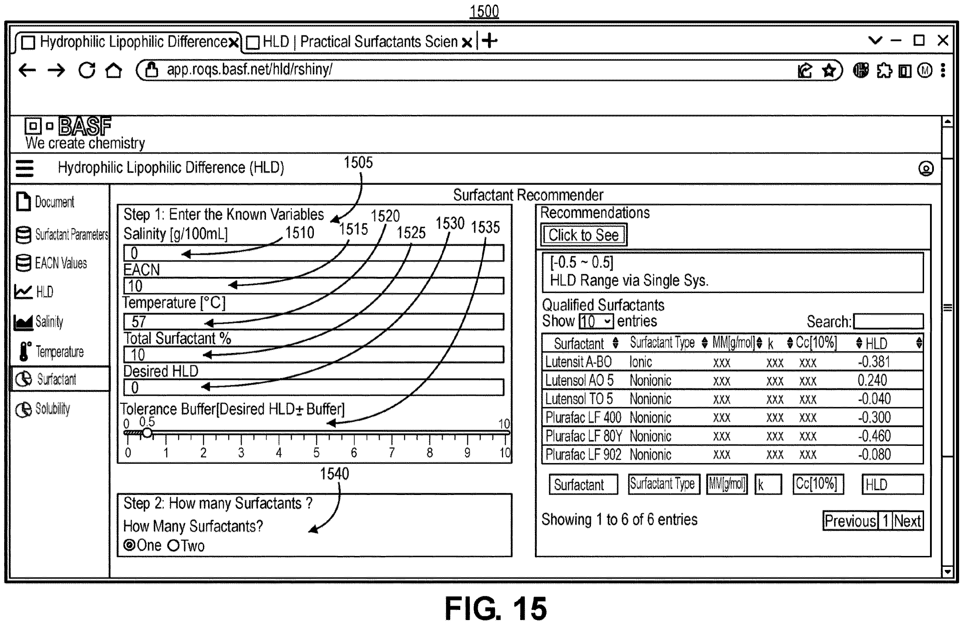

Figure 15 shows an example screen 1500 into which the user 1265 may enter such information. For example, the user 1265 may enter known or desired variables into the entry box 1505. The entered variables may include salinity 1510, EACN 1515, temperature 1520, total surfactant percentage 1525, desired HLD 1530, and tolerance buffer 1535. The user 1265 may further specify a number of surfactants (e.g., to be used in the surfactant mix) into entry box 1540.

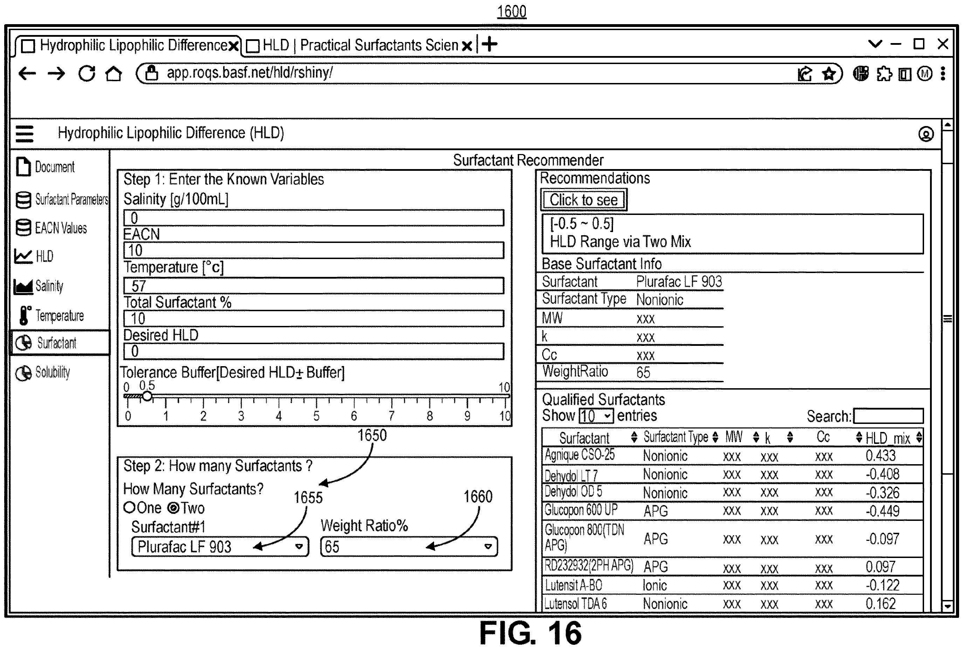

Figure 16 shows an example screen 1600, in which the user has selected the number of surfactants of the surfactant mix to be two. As such, the example screen 1600 also allows the user 1265 to enter a first surfactant 1655, and a weight ratio 1660 of the first surfactant.

The surfactant determining application 1226 may determine the optimal surfactant via any suitable technique. For example, the surfactant determining application 1226 may compare the information entered by the user 1265 to a list of surfactants held in the memory 1222, surfactant database 1218, and/or an external database 1280, as will be described in more detail elsewhere herein.

The surfactant database 1218 may hold any suitable information. For example, the surfactant database 1218 may hold a list of surfactants, and information corresponding to the respective surfactants. For instance, the corresponding information may include surfactant type (e.g., ionic, nonionic, APG, etc.), molar weight, k value, characteristic value $\sigma$, HLD, etc. The corresponding information may also include descriptive information, such as products that the surfactant is known to be used in, known general applications of the surfactant (e.g., a particular surfactant is known to be particularly useful in cleaning motor oil, etc.), etc.

The external database 1280 may also hold any suitable information. For example, the external database 1280 may hold a list of surfactants, and information corresponding to the respective surfactants. For instance, the corresponding information may include surfactant type (e.g., ionic, nonionic, APG, etc.), molar weight, k value, characteristic value $\sigma$, HLD, etc. The corresponding information may also include descriptive information, such as products that the surfactant is known to be used in, known general applications of the surfactant (e.g., a particular surfactant is known to be particularly useful in cleaning motor oil, etc.), etc.

The exemplary computing environment 1200 may further include factory 1290. The factory 1290 may be a factory for producing surfactants, cleaning products, and/or other products. In one working example, the user computing device 1260 sends information to the surfactant computing device 1202, which the surfactant computing device 1202 then uses to determine an optimal surfactant mix. The factory 1290 then produces the optimal surfactant mix and/or a solution including the optimal surfactant mix. The factory 1290 may include surfactant producing device 1295, which may be any suitable device configured to produce, build, or construct surfactants or mixes of surfactants. The factory 1290 may further include solution producing device 1297, which may be any suitable device configured to produce, build, or construct solutions, such as solutions including the surfactant mix.

The factory 1290 may further include a factory computing device 1291, such as a computer, a server, etc. The factory computing device 1291 may perform any functions, such as receiving information (e.g., from the surfactant computing device 1202, the user computing device 1260, etc.), performing calculations (e.g., calculations as described herein with respect to surfactants, solutions, characteristic values, etc.), controlling the surfactant producing device 1295 and/or the solution producing device 1297, etc.

Any of the components in the exemplary computing environment 1200 may communicate via the network 1204 as illustrated. The network 1204 may be a single communication network, or may include multiple communication networks of one or more types (e.g., one or more wired and/or wireless local area networks (LANs), and/or one or more wired and/or wireless wide area networks (WANs), such as the Internet).

Moreover, although the example of Figure 12 illustrates only one of each of many of the components, such as the surfactant computing device 1202, the surfactant database 1218, the external database 1280, the user computing device 1260, the factory 1290, etc., any number of each of the components illustrated in Figure 12 may be included in a system (e.g., multiple user computing devices 1260, multiple factories 1290, multiple surfactant computing devices 1202, etc.).

Example Method For Determining Characteristic Value Of A Nonionic Surfactant Mix Including Two Surfactants

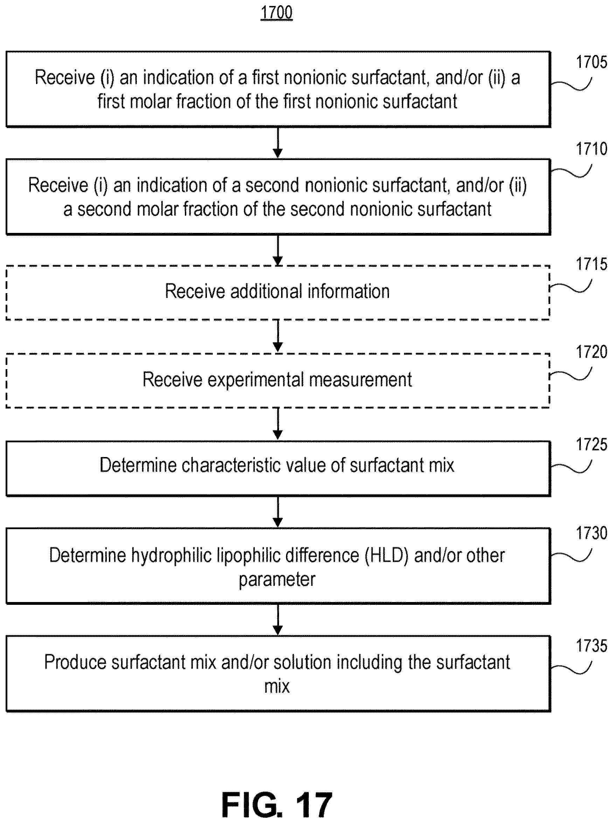

Figure 17 illustrates an example method 1700 for determining a characteristic value of a surfactant mix including two surfactants. In some embodiments, the blocks of the example method 1700 may be performed by the one or more processors 1220. However, it should be understood that any of the blocks may be performed by any suitable component (e.g., the user computing device 1260, factory computing device 1291, etc.).

The example method 1700 begins at block 1705 when the one or more processors 1220 receive (i) an indication of a first nonionic surfactant, and/or (ii) a first molar fraction of the first nonionic surfactant. The one or more processors 1220 may receive this information from any suitable source, such as the user computing device 1260, the factory computing device 1291, etc. The information may have been entered into a screen, such as example screens 1200, 1300, etc.

At block 1710, the one or more processors 1220 receive: (i) an indication of a second nonionic surfactant, and/or (ii) a second molar fraction of the second nonionic surfactant. The one or more processors 1220 may receive this information from any suitable source, such as the user computing device 1260, the factory computing device 1291, etc. The information may have been entered into a screen, such as example screens 1200, 1300, etc.

At optional block 1715, the one or more processors 1220 receive additional information. The additional information may include any information, such as information that the user 1265 enters into entry box 1370. Examples of the additional information include salinity of the solution that the surfactant mix will be part of, EACN of the oil, temperature of the solution, total surfactant percentage in the solution, etc. The one or more processors 1220 may use the additional information for any purpose. In one example, the additional information is used to calculate an HLD of the solution that the surfactant mix will be part of.

At optional block 1720, the one or more processors 1220 receive an experimental measurement. As described above, the experimental measurement is useful in embodiments that use a three-parameter nonlinear equation to determine $\sigma_{mix}$. In particular, in some embodiments, the experimental measurement is used to calculate S of Equation 11. This will be described in further detail below.

At block 1725, the one or more processors 1220 determine a characteristic value of the mix $\sigma_{mix}$. For example, as discussed above, the determination may be made either using a nonlinear equation, such as the two-parameter equation (Equation 10), or the three-parameter equation (Equation 11).

However, as discussed above, it should be understood that that the two-parameter equation is generally less accurate than the three-parameter equation. For example, Figure 5 illustrates that the two-parameter equation curve 510 is generally farther away from the experimental curve 520 than the three-parameter equation curve 530 is. However, as also can be seen from Figure 5, the two-parameter equation curve 510 is more accurate than the linear curve 540. Moreover, advantageously, there is typically enough information (e.g., because the starting and ending points are known because these points correspond to 100% of each individual surfactant) to determine q and r in the two-parameter equation without receiving the experimental measurement (e.g., at block 1720).

It should further be understood that that the three-parameter equation is generally more accurate than the two-parameter equation. In addition to the example of Figure 5, Figures 7 and 8 also illustrate the differences in accuracy. However, to determine the three-parameter equation, additional data, such as that as from the experimental measurement at block 1720, may be required so that all of q, r, and S may be determined.

As this shows, use of either the two-parameter equation or the three-parameter equation improves the technical functioning over systems that use the linear equations. In particular, Figures 5, 7 and 8 all demonstrate the improvement in accuracy over the linear calculation. This improvement further translates into improved solutions (e.g., cleaning or other formulation solutions, etc.) that are made using the techniques described herein. For example, if it is known that a particular HLD cleans a specific oil particularly well, the techniques described herein may be used to produce a surfactant mix that is closer to the desired HLD, thus producing a more effective cleaning formulation solution to clean that specific oil.

Once the nonlinear equation has been determined, the one or more processors 1220 may determine a characteristic value of a surfactant mix $\sigma_{mix}$. The surfactant mix may include the first molar fraction of the first nonionic surfactant, and the second molar fraction of the second nonionic surfactant. The characteristic value may be determined by any suitable technique, such as by inputting, into the nonlinear equation, either the first molar fraction of the first nonionic surfactant or the second molar fraction of the second nonionic surfactant.

At block 1730, the one or more processors 1220 may determine the HLD of the solution that the surfactant mix will be part of. The HLD may be determined by any suitable technique, such as by inputting the $\sigma_{mix}$ determined at block 1725 along with some or all of the additional information received at block 1715 into Equation 1. In some embodiments, the HLD is then used to determine the phase inversion temperature (e.g., the temperature at which an oil-in-water emulsion becomes a water-in-oil emulsion). The phase inversion temperature is useful to know for many technical systems, including systems for ultralow interfacial tension, and systems for emulsion polymerization.

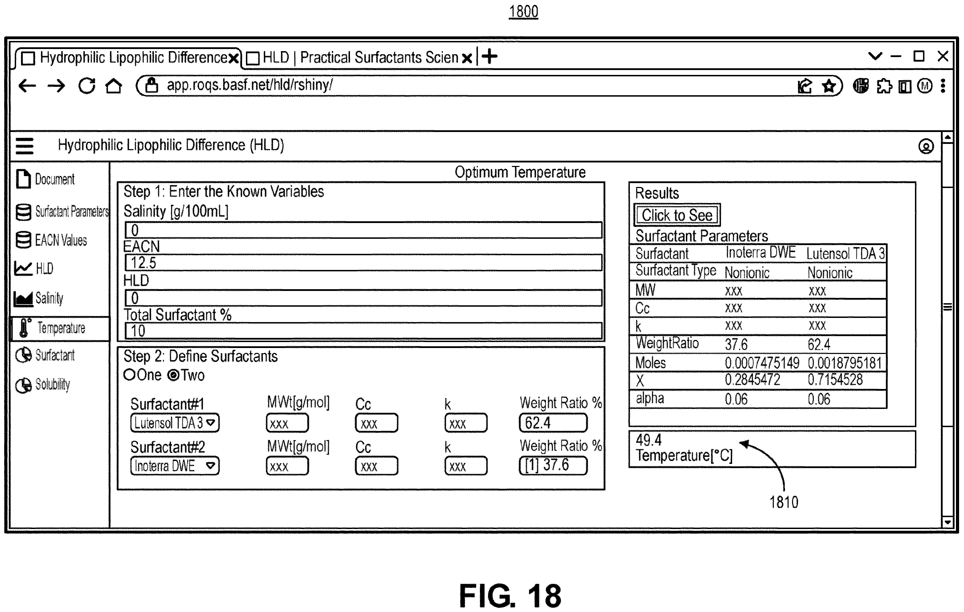

Additionally, or alternatively to calculating the HLD, the one or more processors 1220 may calculate any other parameter. For example, the optimum temperature may be calculated, as in the example screen 1800 of the example of Figure 18, which displays optimum temperature 1810. Additional examples of the parameter include salinity of the solution that the surfactant mix will be part of, EACN of the oil, optimum temperature of the solution, total surfactant percentage in the solution, etc.

The parameter may be determined by any suitable technique, such as by inputting the $\sigma_{mix}$ determined at block

1725 along with some or all of the additional information received at block 1715 into Equation 1. For example, if the parameter is optimum temperature and the one or more processors 1220 have received the salinity, EACN, and HLD from the user computing device 1220 (e.g., at block 1715), Equation 1 may be used to calculate the optimum temperature.

At block 1735, the surfactant mix and/or the solution including the surfactant mix is then produced. For example, the one or more processors 1220 may send information of the surfactant and/or the solution including the surfactant mix to the factory 1290. The factory 1290 may then produce the surfactant mix and/or the solution including the surfactant mix (e.g., via the surfactant producing device 1295 and/or the solution producing device 1297).

Some examples of the solutions include cleaning formulation solutions. For instance, it may be found that a cleaning formulation solution with a particular HLD is better at cleaning motor oil, and another cleaning formulation solution with a different HLD is better at cleaning olive oil (e.g., off of napkins). The principles discussed herein are accordingly useful for each of these applications.

To further elaborate, the cleaning formulation solutions may be used to clean hard surfaces, such as ceramic, stone, marble, glass, plastic, fiberglass, acrylic, stainless steel, painted surfaces, wood. Additionally or alternatively, the cleaning formulation solutions may be used for: floor cleaning; all purpose/multipurpose cleaning; bath, shower, and/or tile cleaning; glass cleaning; kitchen cleaning; household appliance cleaning; furniture cleaning; metal cleaning; membrane cleaning; etc.

Additionally or alternatively, the examples of the solutions may include laundry formulation solutions, such as laundry formulation solutions for: home care laundry; industrial and institutional (I&I) laundry; food and beverage linen laundry; industrial and food processing laundry; healthcare laundry; hospitality laundry; etc.

Additionally or alternatively, the examples of the solutions may include formulation solutions for: cosmetics; oil industry (e.g., enhanced oil recovery, non aqueous drilling fluid removal, etc.); corn oil recovery or demulsification; contamination recovery; emulsion polymerization; mini-emulsion polymerization; agricultural chemical solutions; emulsifiable concentrates; etc.

Example Method For Determining Characteristic Value Of A Nonionic Surfactant Mix Including Three Surfactants

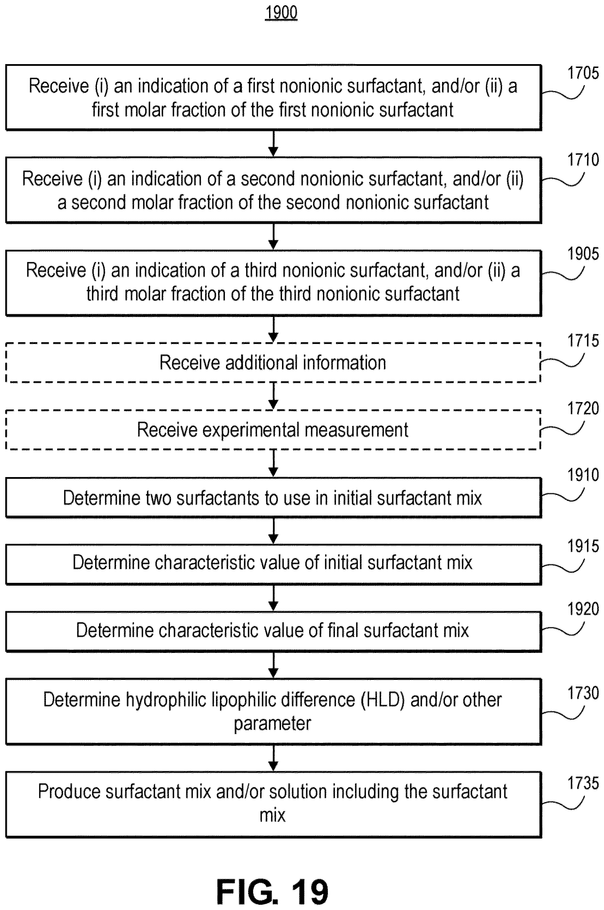

Figure 19 illustrates an example method 1900 for determining a characteristic value of a surfactant mix including three surfactants. By way of overview, when three surfactants are to be mixed, the system may first determine an initial surfactant mix including two of the surfactants; and may then determine a final surfactant mix including the initial surfactant mix and the remaining surfactant.

In some embodiments, the blocks of the example method 1900 may be performed by the one or more processors 1220. However, it should be understood that any of the blocks may be performed by any suitable component (e.g., the user computing device 1260, the factory computing device 1291, etc.). In the example method 1900, blocks 1705 and 1710 may be performed as in the example method 1700.

At block 1905, the one or more processors 1220 receive (i) an indication of a third nonionic surfactant, and/or (ii) a third molar fraction of the third nonionic surfactant. The one or more processors 1220 may receive this information from any suitable source, such as the user computing device 1260, the factory computing device 1291, etc. The information may have been entered into a screen, such as example screens 1200, 1300, etc.

Optional blocks 1715 and 1720 may be performed as in the example method 1700.

At block 1910, the one or more processors 1220 may determine which two of the surfactants to use in an initial surfactant mix. The determination may be made in any suitable way. For example, the three surfactants may be ordered from most hydrophobic to least hydrophobic; the two surfactants to use may then be determined based on the order (e.g., using the two most or least hydrophobic surfactants, or using the most hydrophobic surfactant with the least hydrophobic surfactant). In another example, determination may be made based on molar fractions of the surfactants (e.g., the two surfactants with the largest molar fractions are used). The surfactant determined not to be used in the initial surfactant mix is referred to herein as the "remaining surfactant."

At block 1915, the one or more processors 1220 may determine the characteristic value of the initial surfactant mix $\sigma_{initialmix}$. The characteristic value $\sigma_{initialmix}$ may be determined in any suitable way, such as that described with respect to block 1725, and/or as described elsewhere herein.

At block 1920, the one or more processors 1220 may use $\sigma_{initialmix}$ along with the remaining surfactant to determine the characteristic value of the final surfactant mix $\sigma_{finalmix}$. For example, the one or more processors 1220 may use a nonlinear equation (e.g., the two-parameter equation or the three-parameter equation) with the two surfactants to be mixed being the initial surfactant mix, and the remaining surfactant. In this regard, the characteristic value $\sigma_{finalmix}$ may be determined in any suitable way, such as that described with respect to block 1725, and/or as described elsewhere herein.

Blocks 1730 and 1735 may then be performed as in Figure 17 with the final surfactant mix being the surfactant mix of example method 1700.

Example Method Including Determining To Use Linear Equation(s) Or Nonlinear Equation(s)

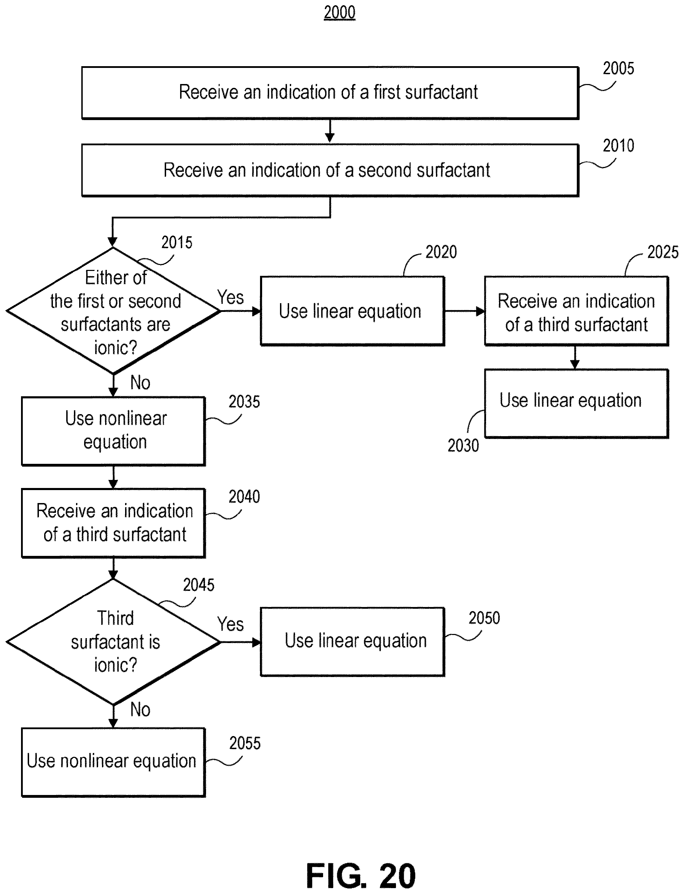

Figure 20 illustrates an example method 2000 including determining to use linear equation(s) or nonlinear equation(s). By way of overview, when surfactants to be mixed are nonionic, a nonlinear equation is used; and, when one of the surfactants to be mixed is ionic, a linear equation is used.

In some embodiments, the blocks of the example method 2000 may be performed by the one or more processors 1220. However, it should be understood that any of the blocks may be performed by any suitable component (e.g., the user computing device 1260, the factory computing device 1291, etc.).

The example method 2000 begins at block 2005 when the one or more processors 1220 receive an indication of a first surfactant. The one or more processors 1220 may receive the indication from any suitable source, such as the user computing device 1260, the factory computing device 1291, etc. The indication may have been entered into a screen, such as example screens 1200, 1300, etc.

At block 2010, the one or more processors 1220 receive an indication of a second surfactant. The one or more processors 1220 may receive the indication from any suitable source, such as the user computing device 1260, the factory computing device 1291, etc. The indication may have been entered into a screen, such as example screens 1200, 1300, etc.

At block 2015, the one or more processors 1220 determine if either of the first or second surfactants are ionic. If so, at block 2020, the one or more processors 1220 use a linear equation to determine the characteristic value $\sigma_{initiaimix}$ of the mix of the first and second surfactants (e.g., according to any of the principles discussed herein, such as in the example of 540 of Figure 5, etc.).

At block 2025, the one or more processors 1220 receive an indication of a third surfactant. The one or more processors 1220 may receive the indication from any suitable source, such as the user computing device 1260, the factory computing device 1291, etc. The indication may have been entered into a screen, such as example screens 1200, 1300, etc.

At block 2030, the one or more processors 1220 determine the characteristic value $\sigma_{finalalmix}$ of the mix of the third surfactant and the surfactant mix determined at block 2020 (e.g., according to any of the principles discussed herein, such as in the example of 540 of Figure 5, etc.).

If, at block 2015, neither of the first or second surfactants is ionic, the one or more processors 1220 use a nonlinear equation to determine the characteristic value $\sigma_{initiaimix}$ of the mix of the first and second surfactants (e.g., using the two-parameter equation or the three-parameter equation) (block 2035).

At block 2040, the one or more processors 1220 receive an indication of a third surfactant. The one or more processors 1220 may receive the indication from any suitable source, such as the user computing device 1260, the factory computing device 1291, etc. The indication may have been entered into a screen, such as example screens 1200, 1300, etc.

At block 2045, the one or more processors 1220 determine if the third surfactant is ionic. If so, at block 2050, the one or more processors 1220 use a linear equation to determine the characteristic value $\sigma_{finalmix}$ of the mix of the third surfactant and surfactant mix determined at block 2035 (e.g., according to any of the principles discussed herein, such as in the example of 540 of Figure 5, etc.).

If, at block 2045, the third surfactant is not ionic, the one or more processors 1220 use a nonlinear equation to determine the characteristic value $\sigma_{finalmix}$ of the mix of the third surfactant and the surfactant mix determined at block 2035 (e.g., using the two-parameter equation or the three-parameter equation) (block 2055).

Example Method For Determining an Optimal Surfactant

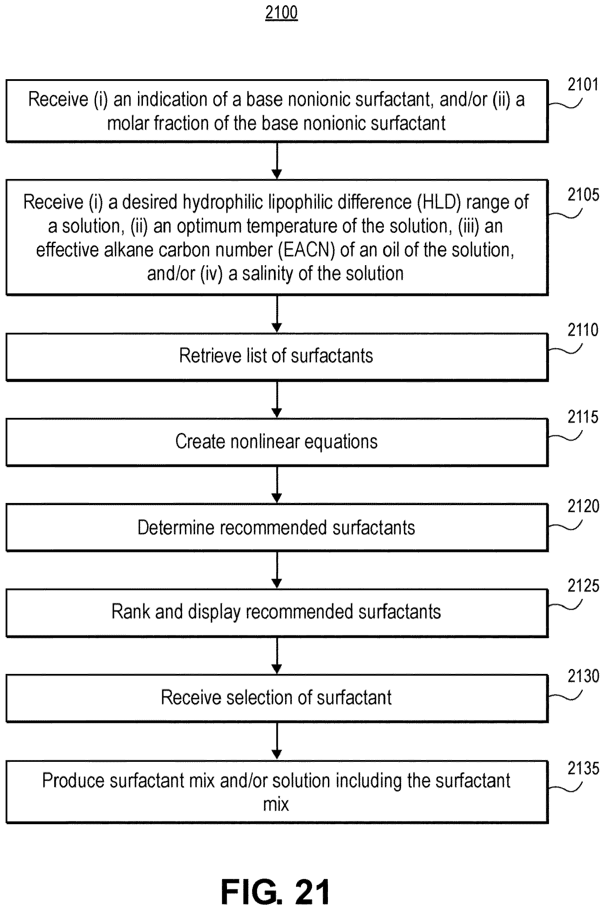

Figure 21 illustrates an example method 2100 for determining an optimal surfactant.

In some embodiments, the blocks of the example method 2100 may be performed by the one or more processors 1220. However, it should be understood that any of the blocks may be performed by any suitable component (e.g., the user computing device 1260, the factory computing device 1291, etc.).

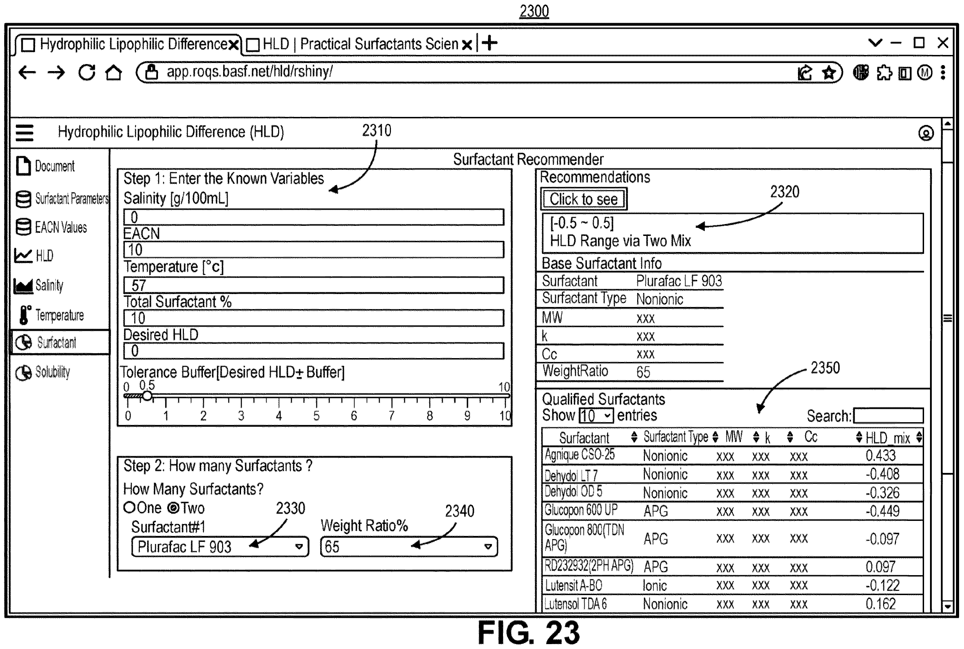

The example method 2100 begins at block 2101 when the one or more processors 1220 receive (i) an indication of a base nonionic surfactant, and/or (ii) a molar fraction of the base nonionic surfactant. The one or more processors 1220 may receive this information from any suitable source, such as the user computing device 1260, the factory computing device 1291, etc. The information may have been entered into a screen, such as example screen 2300 of Figure 23 (e.g., via entry box 2330).

At block 2105, the one or more processors 1220 receive: (i) a desired hydrophilic lipophilic difference (HLD) range of a solution, (ii) an optimum temperature of the solution, (iii) an effective alkane carbon number (EACN) of an oil of the solution, and/or (iv) a salinity of the solution. The one or more processors 1220 may receive this information from any suitable source, such as the user computing device 1260, the factory computing device 1291, etc.

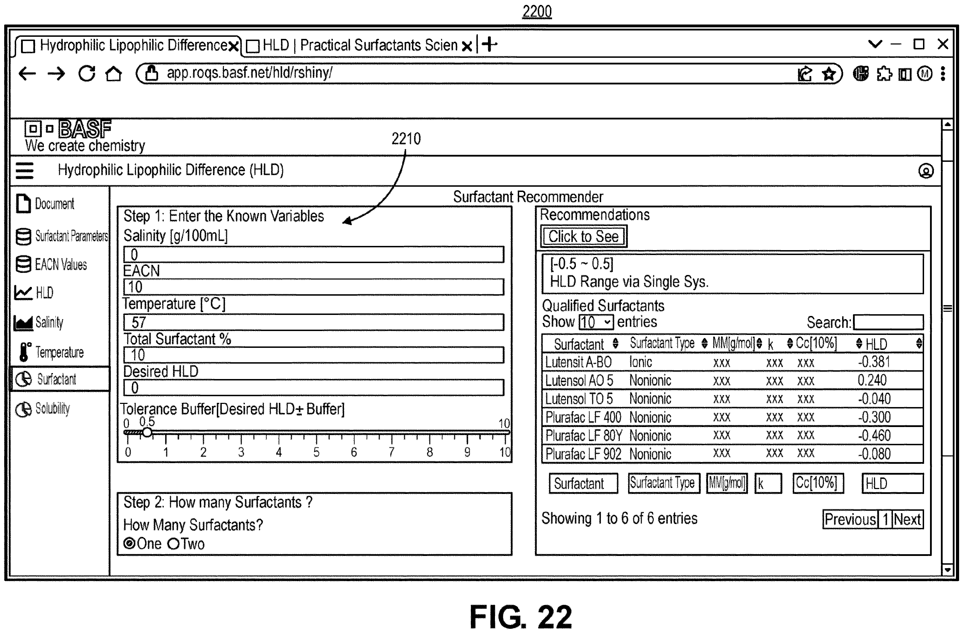

In some examples, the user 1265 enters this information to be sent to the one or more processors 1220 into a screen of the user computing device 1260, such as the example screen 2200 of the example of Figure 22. For example, a salinity, an EACN, a temperature, a total surfactant percentage, a desired HLD, and/or a tolerance buffer for the desired HDL may be entered. It should thus be understood that by entering the desired HLD and the tolerance buffer for the desired HLD, an HLD range is entered.

Figure 23 shows an additional example screen 2300 into which the information may be entered (e.g., via entry box 2310). More specifically, Figure 23 illustrates an example of determining a single surfactant, whereas Figure 22 illustrates an example for determining a surfactant for a surfactant mix comprising two surfactants.

At block 2110, the one or more processors 1220, retrieve a list of surfactants. The list of surfactants may be retrieved from any suitable source, such as from the surfactant database 1218, the external database 1280, and/or the memory 1222.

At block 2115, the one or more processors 1220 create nonlinear equations for respective nonionic surfactant mixes. The nonlinear equations may: (i) correspond, respectively, to nonionic surfactants in the list of surfactants, and (ii) be based on (a) the base nonionic surfactant and (b) the molar fraction of the base nonionic surfactant. The nonlinear equations may be created as described herein; for example, the non-linear equations may be created using the two-parameter nonlinear equation and/or the three-parameter nonlinear equation (if enough data is available to determine a three-parameter equation for a particular surfactant mix).

At block 2120, the one or more processors 1220 may determine at least one recommended surfactant. The determination may be made by any suitable technique. For example, the determination may be made based on: (i) the desired HLD range (e.g., received at block 2105), and/or (ii) the created nonlinear equations.

In one working example, the desired HLD range is -0.5-0.5 (e.g., as in the example screen 2300 including HLD range 2320), and the entered base surfactant 2330 is Plurafac LF 903. The one or more processors 1220 determine a series of nonlinear equations for respective combinations of Plurafac LF 903 with other surfactants from the list of surfactants. Then, using the molar weight/weight ratio % (e.g., as entered in entry box 2340 of Figure 23) and the respective nonlinear equations, the one or more processors 1220 determine the recommended surfactants (e.g., surfactants that create surfactant mixes with HLDs falling within the desired HLD range).

It should be further understood that the HLDs may be further calculated according to Equation 1 (e.g., based on the salinity, EACN, and/or temperature received at block 2105).

Examples of surfactants that may be recommended are: Agnique CSO-25; Agnique CSO-36; Lutensol AO 3; Lutensol AO 5; Lutensol AO 7; Lutensol AO 8; Lutensol AO 11; Lutensol LA60; Lutensol TDA3; Lutensol TDA6; Lutensol TDA8; Lutensol TDA9; Lutensol TDA 10; Lutensol TO 2; Lutensol TO 3; Lutensol TO 5; Lutensol TO 6; Lutensol TO 7; Lutensol TO 8;; Lutensol XL 70; Lutensol XL 90; SLS Texapon or Standapol products; Inoterra DWE; Lutensit A-BO; Lutensol TO 5; Plurafac LF 400; Plurafac LF 80Y; Plurafac LF 902; Agnique CS0-25; Dehydol LT7; Dehydol OD5; Glucopon 800; Glucopon 600 UP, Glucopon 215 UP, Glucopon 420 UP, Glucopon 325 N; Dehyton PK-45, PL, etc.

At block 2125, the one or more processors 1220 rank and display the recommended surfactants, such as in ranked surfactant list 2350. The recommended surfactants may be ranked in any suitable way. For example, they may be ranked according to their corresponding surfactant mix's proximity to the desired HLD. Alternatively, they may be ranked according to their molar weight, their k value, or their characteristic value $\sigma_{mix}$.

At block 2130, the user 1265 selects a surfactant (e.g., via the user computing device 1260). The selection may then be sent to factory 1290, which may produce the selected surfactant, the corresponding surfactant mix, and/or a solution including the surfactant mix.

Further regarding the example flowcharts provided above, it should be noted that all blocks are not necessarily required to be performed. Moreover, additional blocks may be performed although they are not specifically illustrated in the example flowcharts. In addition, the example flowcharts are not mutually exclusive. For example, block(s) from one example flowchart may be performed in another of the example flowcharts. In addition, not all blocks in each flowchart are required to be performed.


Additional Embodiments


Aspect 1. A computer-implemented method for determining a characteristic value of a nonionic surfactant mix, the method comprising:

> receiving, via one or more processors: (i) an indication of a first nonionic surfactant, and (ii) a first molar fraction of the first nonionic surfactant;
> receiving, via the one or more processors: (i) an indication of a second nonionic surfactant, and (ii) a second molar fraction of the second nonionic surfactant; and
> determining, via the one or more processors, a characteristic value of a surfactant mix including the first molar fraction of the first nonionic surfactant, and the second molar fraction of the second nonionic surfactant, and wherein the characteristic value is determined by inputting, into a nonlinear equation, either the first molar fraction of the first nonionic surfactant or the second molar fraction of the second nonionic surfactant.

Aspect 2. The computer-implemented method of aspect 1, wherein:

the nonlinear equation comprises a two-parameter nonlinear equation;
the determining the characteristic value comprises inputting the first molar fraction of the first nonionic surfactant into the two-parameter nonlinear equation; and
the method further comprises determining first and second parameters of the two-parameter nonlinear equation based on the first nonionic surfactant.

Aspect 3. The computer-implemented method of aspect 2, wherein the two-parameter nonlinear equation comprises:

$$\sigma_{mix} = q + re^{\chi_1}$$

where:

$\sigma_{mix}$ is the characteristic value;
q is the first parameter;
r is the second parameter; and
$\chi_1$ is the first molar fraction of the first nonionic surfactant.

Aspect 4. The computer-implemented method of any one of aspects 1-3, wherein:

the nonlinear equation comprises a three-parameter nonlinear equation;
the determining the characteristic value comprises inputting the first molar fraction of the first nonionic surfactant into the three-parameter nonlinear equation; and
the method further comprises determining first and second parameters of the three-parameter nonlinear equation based on the first nonionic surfactant and the second nonionic surfactant.

Aspect 5. The computer-implemented method of any one of aspects 1-4, wherein the third parameter is determined by receiving an input based on an experimental measurement.
Aspect 6. The computer-implemented method of any one of aspects 1-5, wherein the three-parameter nonlinear equation comprises:

$$\sigma_{mix} = q + re^{s\chi_1}$$

where:

$\sigma_{mix}$ is the characteristic curvature;
q is the first parameter;
r is the second parameter;
s is the third parameter; and
$\chi_1$ is the first molar fraction of the first nonionic surfactant.

Aspect 7. The computer-implemented method of any one of aspects 1-6, further comprising:

determining, via the one or more processors, a hydrophilic lipophilic difference (HLD) of the surfactant mix based on the determined characteristic value; and determining, via the one or more processors, a formulation solution based on the determined HLD; and
wherein the formulation solution includes:

a corn oil demulsification formulation solution;
an emulsion polymerization formulation solution; or
an Agricultural chemical benefit formulation solution.

Aspect 8. The computer-implemented method of any one of aspects 1-7, wherein the surfactant mix is a first surfactant mix and the linear equation is a first nonlinear equation, and wherein the method further comprises:

receiving, via the one or more processors: (i) an indication of a third nonionic surfactant, and (ii) a third molar fraction of the third nonionic surfactant; and
determining, via the one or more processors, a characteristic value of a second surfactant mix including a molar fraction of the first surfactant mix, and the third molar fraction of the third nonionic surfactant, and wherein the characteristic value of the second surfactant mix is determined by inputting, into a second nonlinear equation, either the molar fraction of the first surfactant mix or the molar fraction of the third nonionic surfactant.

Aspect 9. The computer-implemented method of any one of aspects 1-8, wherein the surfactant mix is a first surfactant mix, and wherein the method further comprises:

receiving, via one or more processors: (i) an indication of an ionic surfactant, and (ii) a molar fraction of the ionic surfactant; and
determining, via the one or more processors, a characteristic value of a second surfactant mix including a molar fraction of the first surfactant mix, and the molar fraction of the ionic surfactant, and wherein the characteristic value of the second surfactant mix is determined by inputting, into a linear equation, either the molar fraction of the first surfactant mix or the molar fraction of the ionic surfactant.

Aspect 10. A computer-implemented method for determining an optimal surfactant, the method comprising:

receiving, via one or more processors: (i) an indication of a base nonionic surfactant, and (ii) a molar fraction of the base nonionic surfactant;
receiving, via the one or more processors, a desired hydrophilic lipophilic difference (HLD) range of a solution including a desired surfactant mix comprising the base nonionic surfactant;
retrieving, via the one or more processors, a list of surfactants, from a surfactant database; and
determining, via the one or more processors, at least one recommended surfactant by:

creating nonlinear equations, wherein the nonlinear equations: (i) correspond, respectively, to nonionic surfactants in the list of surfactants, and (ii) are based on (a) the base nonionic surfactant and (b) the molar fraction of the base nonionic surfactant; and
determining the at least one recommended surfactant based on: (i) the desired HLD range, and (ii) the created nonlinear equations.

Aspect 11. The computer-implemented method of aspect 10, wherein:

the solution further comprises an oil and a water;
the method further comprises receiving, via the one or more processors, (i) a temperature of the surfactant mix, (ii) an effective alkane carbon number (EACN) of the oil, and (iii) a salinity of the solution; and
the at least one recommended surfactant is determined further based on: (i) the temperature of the surfactant mix, (ii) the EACN of the oil, and (iii) the salinity of the solution.

Aspect 12. The computer-implemented method of any one of aspects 10-11, wherein the one or more processors receive the desired HLD range of solution by receiving (i) a desired HLD number, and (ii) a tolerance buffer.

Aspect 13. The computer-implemented method of any one of aspects 10-12, wherein the at least one recommended surfactant comprises a plurality of recommended surfactants, and the method further comprises:

ranking, via the one or more processors, recommended surfactants of the plurality of recommended surfactants according to the desired HLD number; and
displaying, via the one or more processors, the ranked recommended surfactants on a display.

Aspect 14. The computer-implemented method of any one of aspects 10-14, further comprising producing the recommended surfactant.

Aspect 15. A computer system for determining a characteristic value of a nonionic surfactant mix, the computer system comprising one or more processors configured to:

receive: (i) an indication of a first nonionic surfactant, and (ii) a first molar fraction of the first nonionic surfactant;

receive: (i) an indication of a second nonionic surfactant, and (ii) a second molar fraction of the second nonionic surfactant; and

determine, a characteristic value of a surfactant mix including the first molar fraction of the first nonionic surfactant, and the second molar fraction of the second nonionic surfactant, and wherein the characteristic value is determined by inputting, into a nonlinear equation, either the first molar fraction of the first nonionic surfactant or the second molar fraction of the second nonionic surfactant.

Aspect 16. The computer system of aspect 15, wherein:

the nonlinear equation comprises a two-parameter nonlinear equation; and
the one or more processors are further configured to determine the characteristic value by inputting the first molar fraction of the first nonionic surfactant into the two-parameter nonlinear equation.

Aspect 17. The computer system of any one of aspects 15-16, wherein:

the nonlinear equation comprises a three-parameter nonlinear equation;
the one or more processors are further configured to determine the characteristic value by inputting the first molar fraction of the first nonionic surfactant into the three-parameter nonlinear equation; and
the one or more processors are further configured to determine first and second parameters of the three-parameter nonlinear equation based on the first nonionic surfactant and the second nonionic surfactant.

Aspect 18. The computer system of any one of aspects 15-17, wherein the one or more processors are further configured to determine the third parameter by receiving an input based on an experimental measurement.
Aspect 19. The computer system of any one of aspects 15-18, one or more processors are further configured to determine:

a hydrophilic lipophilic difference (HLD) of the surfactant mix based on the determined characteristic value; and
a cleaning formulation solution based on the determined HLD.

Aspect 20. The computer system of any one of aspects 15-19, wherein the surfactant mix is a first surfactant mix and the linear equation is a first nonlinear equation, and wherein the one or more processors are further configured to:

receive: (i) an indication of a third nonionic surfactant, and (ii) a third molar fraction of the third nonionic surfactant; and
determine a characteristic value of a second surfactant mix including a molar fraction of the first surfactant mix, and the third molar fraction of the third nonionic surfactant, and wherein the characteristic value of the second surfactant mix is determined by inputting, into a second nonlinear equation, either the molar fraction of the first surfactant mix or the molar fraction of the third nonionic surfactant.

Other Matters

Additionally, certain embodiments are described herein as including logic or a number of routines, subroutines, applications, or instructions. These may constitute either software (code embodied on a non-transitory, tangible machine-readable medium) or hardware. In hardware, the routines, etc., are tangible units capable of performing certain operations and may be configured or arranged in a certain manner. In example embodiments, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.
In various embodiments, a hardware module may be implemented mechanically or electronically. For example, a hardware module may comprise dedicated circuitry or logic that is permanently configured (e.g., as a special-purpose processor, such as a field programmable gate array (FPGA) or an application-specific integrated circuit (ASIC) to perform certain operations. A hardware module may also comprise programmable logic or circuitry (e.g., as encompassed within a general-purpose processor or other programmable processor) that is temporarily configured by software to perform certain operations. It will be appreciated that the decision to implement a hardware module mechanically, in dedicated and permanently configured circuitry, or in temporarily configured circuitry (e.g., configured by software) may be driven by cost and time considerations.
Accordingly, the term "hardware module" should be understood to encompass a tangible entity, be that an entity that is physically constructed, permanently configured (e.g., hardwired), or temporarily configured (e.g., programmed) to operate in a certain manner or to perform certain operations described herein. Considering embodiments in which hardware modules are temporarily configured (e.g., programmed), each of the hardware modules need not be configured or instantiated at any one instance in time. For example, where the hardware modules comprise a general-purpose processor configured using software, the general-purpose processor may be configured as respective different hardware modules at different times. Software may accordingly configure a processor, for example, to constitute a particular hardware module at one instance of time and to constitute a different hardware module at a different instance of time.

Hardware modules can provide information to, and receive information from, other hardware modules. Accordingly, the described hardware modules may be regarded as being communicatively coupled. Where multiple of such hardware modules exist contemporaneously, communications may be achieved through signal transmission (e.g., over appropriate circuits and buses) that connect the hardware modules. In embodiments in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and can operate on a resource (e.g., a collection of information).

The various operations of example methods described herein may be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example embodiments, comprise processor-implemented modules.

Similarly, the methods or routines described herein may be at least partially processor-implemented. For example, at least some of the operations of a method may be performed by one or more processors or processor-implemented hardware modules. The performance of certain of the operations may be distributed among the one or more processors, not only residing within a single machine, but deployed across a number of machines. In some example embodiments, the processor or processors may be located in a single location (e.g., within a home environment, an office environment or as a server farm), while in other embodiments the processors may be distributed across a number of geographic locations.

Furthermore, the patent claims at the end of this patent application are not intended to be construed under 35 U.S.C. § 112(f) unless traditional means-plus-function language is expressly recited, such as "means for" or "step for" language being explicitly recited in the claim(s). The systems and methods described herein are directed to an improvement to computer functionality, and improve the functioning of conventional computers.

**Claims**

1. A computer-implemented method for determining a characteristic value of a nonionic surfactant mix, the method comprising:

   receiving, via one or more processors: (i) an indication of a first nonionic surfactant, and (ii) a first molar fraction of the first nonionic surfactant;
   receiving, via the one or more processors: (i) an indication of a second nonionic surfactant, and (ii) a second molar fraction of the second nonionic surfactant; and
   determining, via the one or more processors, a characteristic value of a surfactant mix including the first molar fraction of the first nonionic surfactant, and the second molar fraction of the second nonionic surfactant, and wherein the characteristic value is determined by inputting, into a nonlinear equation, either the first molar fraction of the first nonionic surfactant or the second molar fraction of the second nonionic surfactant.

2. The computer-implemented method of claim 1, wherein:

   the nonlinear equation comprises a two-parameter nonlinear equation;
   the determining the characteristic value comprises inputting the first molar fraction of the first nonionic surfactant into the two-parameter nonlinear equation; and
   the method further comprises determining first and second parameters of the two-parameter nonlinear equation based on the first nonionic surfactant.

3. The computer-implemented method of claim 2, wherein the two-parameter nonlinear equation comprises:

$$\sigma_{mix} = q + re^{\chi_1}$$

where:

$\sigma_{mix}$ is the characteristic value;
q is the first parameter;
r is the second parameter; and
$\chi_1$ is the first molar fraction of the first nonionic surfactant.

4. The computer-implemented method of claim 1, wherein:

the nonlinear equation comprises a three-parameter nonlinear equation;
the determining the characteristic value comprises inputting the first molar fraction of the first nonionic surfactant into the three-parameter nonlinear equation; and
the method further comprises determining first and second parameters of the three-parameter nonlinear equation based on the first nonionic surfactant and the second nonionic surfactant.

5. The computer-implemented method of claim 4, wherein the third parameter is determined by receiving an input based on an experimental measurement.

6. The computer-implemented method of claim 5, wherein the three-parameter nonlinear equation comprises:

$$\sigma_{mix} = q + re^{s\chi_1}$$

where:

$\sigma_{mix}$ is the characteristic curvature;
q is the first parameter;
r is the second parameter;
s is the third parameter; and
$\chi_1$ is the first molar fraction of the first nonionic surfactant.

7. The computer-implemented method of claim 1, further comprising:

determining, via the one or more processors, a hydrophilic lipophilic difference (HLD) of the surfactant mix based on the determined characteristic value; and
determining, via the one or more processors, a formulation solution based on the determined HLD; and
wherein the formulation solution includes:

a corn oil demulsification formulation solution;
an emulsion polymerization formulation solution; or
an Agricultural chemical benefit formulation solution.

8. The computer-implemented method of claim 1, wherein the surfactant mix is a first surfactant mix and the linear equation is a first nonlinear equation, and wherein the method further comprises:

receiving, via the one or more processors: (i) an indication of a third nonionic surfactant, and (ii) a third molar fraction of the third nonionic surfactant; and
determining, via the one or more processors, a characteristic value of a second surfactant mix including a molar fraction of the first surfactant mix, and the third molar fraction of the third nonionic surfactant, and wherein the characteristic value of the second surfactant mix is determined by inputting, into a second nonlinear equation, either the molar fraction of the first surfactant mix or the molar fraction of the third nonionic surfactant.

9. The computer-implemented method of claim 1, wherein the surfactant mix is a first surfactant mix, and wherein the method further comprises:

receiving, via one or more processors: (i) an indication of an ionic surfactant, and (ii) a molar fraction of the ionic surfactant; and

determining, via the one or more processors, a characteristic value of a second surfactant mix including a molar fraction of the first surfactant mix, and the molar fraction of the ionic surfactant, and wherein the characteristic value of the second surfactant mix is determined by inputting, into a linear equation, either the molar fraction of the first surfactant mix or the molar fraction of the ionic surfactant.

10. A computer-implemented method for determining an optimal surfactant, the method comprising:

receiving, via one or more processors: (i) an indication of a base nonionic surfactant, and (ii) a molar fraction of the base nonionic surfactant;

receiving, via the one or more processors, a desired hydrophilic lipophilic difference (HLD) range of a solution including a desired surfactant mix comprising the base nonionic surfactant;

retrieving, via the one or more processors, a list of surfactants, from a surfactant database; and

determining, via the one or more processors, at least one recommended surfactant by:

creating nonlinear equations, wherein the nonlinear equations: (i) correspond, respectively, to nonionic surfactants in the list of surfactants, and (ii) are based on (a) the base nonionic surfactant and (b) the molar fraction of the base nonionic surfactant; and

determining the at least one recommended surfactant based on: (i) the desired HLD range, and (ii) the created nonlinear equations.

11. The computer-implemented method of claim 10, wherein:

the solution further comprises an oil and a water;

the method further comprises receiving, via the one or more processors, (i) a temperature of the surfactant mix, (ii) an effective alkane carbon number (EACN) of the oil, and (iii) a salinity of the solution; and

the at least one recommended surfactant is determined further based on: (i) the temperature of the surfactant mix, (ii) the EACN of the oil, and (iii) the salinity of the solution.

12. The computer-implemented method of claim 10, wherein the one or more processors receive the desired HLD range of solution by receiving (i) a desired HLD number, and (ii) a tolerance buffer.

13. The computer-implemented method of claim 12, wherein the at least one recommended surfactant comprises a plurality of recommended surfactants, and the method further comprises:

ranking, via the one or more processors, recommended surfactants of the plurality of recommended surfactants according to the desired HLD number; and

displaying, via the one or more processors, the ranked recommended surfactants on a display.

14. The computer-implemented method of claim 10, further comprising producing the recommended surfactant.

15. A computer system for determining a characteristic value of a nonionic surfactant mix, the computer system comprising one or more processors configured to:

receive: (i) an indication of a first nonionic surfactant, and (ii) a first molar fraction of the first nonionic surfactant;

receive: (i) an indication of a second nonionic surfactant, and (ii) a second molar fraction of the second nonionic surfactant; and

determine, a characteristic value of a surfactant mix including the first molar fraction of the first nonionic surfactant, and the second molar fraction of the second nonionic surfactant, and wherein the characteristic value is determined by inputting, into a nonlinear equation, either the first molar fraction of the first nonionic surfactant or the second molar fraction of the second nonionic surfactant.

16. The computer system of claim 15, wherein:

the nonlinear equation comprises a two-parameter nonlinear equation; and

the one or more processors are further configured to determine the characteristic value by inputting the first molar fraction of the first nonionic surfactant into the two-parameter nonlinear equation.

**17.** The computer system of claim 15, wherein:

the nonlinear equation comprises a three-parameter nonlinear equation;
the one or more processors are further configured to determine the characteristic value by inputting the first molar fraction of the first nonionic surfactant into the three-parameter nonlinear equation; and
the one or more processors are further configured to determine first and second parameters of the three-parameter nonlinear equation based on the first nonionic surfactant and the second nonionic surfactant.

**18.** The computer system of claim 15, wherein the one or more processors are further configured to determine the third parameter by receiving an input based on an experimental measurement.

**19.** The computer system of claim 15, one or more processors are further configured to determine:

a hydrophilic lipophilic difference (HLD) of the surfactant mix based on the determined characteristic value; and
a cleaning formulation solution based on the determined HLD.

**20.** The computer system of claim 15, wherein the surfactant mix is a first surfactant mix and the linear equation is a first nonlinear equation, and wherein the one or more processors are further configured to:

receive: (i) an indication of a third nonionic surfactant, and (ii) a third molar fraction of the third nonionic surfactant; and
determine a characteristic value of a second surfactant mix including a molar fraction of the first surfactant mix, and the third molar fraction of the third nonionic surfactant, and wherein the characteristic value of the second surfactant mix is determined by inputting, into a second nonlinear equation, either the molar fraction of the first surfactant mix or the molar fraction of the third nonionic surfactant.

## f(S)+c_T(T-25) vs. EACN

$Y = 0.8025 + 0.106*EACN$
$R^2$: 0.999

**FIG. 1**

## Predicted and Experimental σ Values vs. Mole% TO 3

**FIG. 2**

**Predicted and Experimental σ Values vs. Mole% TO 3**

**FIG. 3**

**Experimental σ_mix vs. Mole% TO 3**

**FIG. 4**

## Predicted and Experimental σ Values vs. Mole% TO 3

**FIG. 5**

## Predicted and Experimental σ Values vs. Mole% TO 3

**FIG. 6**

**Predicted and Experimental σ_mix vs. Mole% TO 3**

FIG. 7

**Predicted and Experimental S* vs. Mole% TO 7**

FIG. 8

**Predicted σ_mix vs. Mole% TO 3**

FIG. 9

**Predicted σ_mix vs. Mole% TO 3**

FIG. 10

Predicted σ_mix vs. Mole% TO3

Predicted σ_mix [linear]
Predicted σ_mix [Exp2P, TO3]
Predicted σ_mix [Exp2P, AO3]
* Experimental σ_mix

FIG. 11

1200

SURFACTANT COMPUTING
DEVICE
1202

PROCESSOR(S)
1220

MEMORY
1222

CHARACTERISTIC VALUE
APPLICATION
1224

SURFACTANT DETERMINING
APPLICATION
1226

SURFACTANT
DATABASE
1218

EXTERNAL
DATABASE
1280

NETWORK
1204

FACTORY
1290

FACTORY
COMPUTING
DEVICE
1291

SURFACTANT
PRODUCING
DEVICE
1295

SOLUTION
PRODUCING
DEVICE
1297

USER COMPUTING
DEVICE
1260

USER
1265

FIG. 12

EP 4 451 279 A1

1300

**FIG. 13**

EP 4 451 279 A1

1400

EP 4 451 279 A1

Hydrophilic Lipophilic Difference ✕ | HLD | Practical Surfactants Scien ✕ | +

← → C ⌂  app.roqs.basf.net/hld/rshiny/

☰  Hydrophilic Lipophilic Difference (HLD)    1460

**HLD**

Document
Surfactant Parameters
EACN Values
HLD
Salinity
Temperature
Surfactant
Solubility

**Step 1: Adjust Variables**

Salinity [g/100mL]
| 1 |

EACN
| 8 |

Temperature [°c]
| 37 |

Total Surfactant %
| 10 |

**Step 2: How Many Surfactants?**
○ One  ○ Two  ◉ Three

**Step 3: Select Surfactants**

| Surfactant#1 | MW | Cc | k | Weight Ratio % |
| Lutensol AO5 ▾ | xxx | xxx | xxx | 10 |

| Surfactant#2 | MW | Cc | k | Weight Ratio % |
| Lutensol TDA 6 ▾ | xxx | xxx | xxx | 20 |

| Surfactant#3 | MW | Cc | k | Weight Ratio % |
| Lutensol XL 90 ▾ | xxx | xxx | xxx | [1] 70 |

**Results**

| Click to See |

**Surfactant Parameters**

| Surfactant | Lutensol AO 5 | Lutensol TDA 6 | Lutensol XL 90 |
| --- | --- | --- | --- |
| Surfactant Type | Nonionic | Nonionic | Nonionic |
| MW | xxx | xxx | xxx |
| Cc | xxx | xxx | xxx |
| k | xxx | xxx | xxx |
| WeightRatio | 10 | 20 | 70 |
| Moles | 0.0002314815 | 0.0004273504 | 0.0011075949 |
| X | 0.1310450 | 0.2419293 | 0.6270256 |
| alpha | 0.06 | 0.06 | 0.06 |

-2.91
HLD(Nonelinear, Mixed by 3 Nonionic Surfactants)

0.169
$K_{mix}$

0.06
$alpha_{mix}$

-2.402
$Cc_{mix}$ (Nonlinear, 3 Nonionic Surfactants)

**FIG. 14**

FIG. 15

EP 4 451 279 A1

**FIG. 16**

<u>1700</u>

```
┌─────────────────────────────────────────────────────────┐
│ Receive (i) an indication of a first nonionic surfactant,│  1705
│ and/or (ii) a first molar fraction of the first nonionic │
│ surfactant                                               │
└─────────────────────────────────────────────────────────┘
                            │
                            ▼
┌─────────────────────────────────────────────────────────┐
│ Receive (i) an indication of a second nonionic surfactant,│ 1710
│ and/or (ii) a second molar fraction of the second        │
│ nonionic surfactant                                      │
└─────────────────────────────────────────────────────────┘
                            │
                            ▼
┌ ─ ─ ─ ─ ─ ─ ─ ─ ─ ─ ─ ─ ─ ─ ─ ─ ─ ─ ─ ─ ─ ─ ─ ─ ─ ─ ─ ┐
    Receive additional information                           1715
└ ─ ─ ─ ─ ─ ─ ─ ─ ─ ─ ─ ─ ─ ─ ─ ─ ─ ─ ─ ─ ─ ─ ─ ─ ─ ─ ─ ┘
                            │
                            ▼
┌ ─ ─ ─ ─ ─ ─ ─ ─ ─ ─ ─ ─ ─ ─ ─ ─ ─ ─ ─ ─ ─ ─ ─ ─ ─ ─ ─ ┐
    Receive experimental measurement                         1720
└ ─ ─ ─ ─ ─ ─ ─ ─ ─ ─ ─ ─ ─ ─ ─ ─ ─ ─ ─ ─ ─ ─ ─ ─ ─ ─ ─ ┘
                            │
                            ▼
┌─────────────────────────────────────────────────────────┐
│ Determine characteristic value of surfactant mix         │  1725
└─────────────────────────────────────────────────────────┘
                            │
                            ▼
┌─────────────────────────────────────────────────────────┐
│ Determine hydrophilic lipophilic difference (HLD) and/or │  1730
│ other parameter                                          │
└─────────────────────────────────────────────────────────┘
                            │
                            ▼
┌─────────────────────────────────────────────────────────┐
│ Produce surfactant mix and/or solution including the     │  1735
│ surfactant mix                                           │
└─────────────────────────────────────────────────────────┘
```

# FIG. 17

FIG. 18

1900

| Receive (i) an indication of a first nonionic surfactant, and/or (ii) a first molar fraction of the first nonionic surfactant | 1705 |

| Receive (i) an indication of a second nonionic surfactant, and/or (ii) a second molar fraction of the second nonionic surfactant | 1710 |

| Receive (i) an indication of a third nonionic surfactant, and/or (ii) a third molar fraction of the third nonionic surfactant | 1905 |

| Receive additional information | 1715 |

| Receive experimental measurement | 1720 |

| Determine two surfactants to use in initial surfactant mix | 1910 |

| Determine characteristic value of initial surfactant mix | 1915 |

| Determine characteristic value of final surfactant mix | 1920 |

| Determine hydrophilic lipophilic difference (HLD) and/or other parameter | 1730 |

| Produce surfactant mix and/or solution including the surfactant mix | 1735 |

# FIG. 19

2000

Receive an indication of a first surfactant — 2005

Receive an indication of a second surfactant — 2010

2015
Either of the first or second surfactants are ionic?

Yes → Use linear equation — 2020 → Receive an indication of a third surfactant — 2025

Use linear equation

2030

No

Use nonlinear equation — 2035

Receive an indication of a third surfactant — 2040

2045
Third surfactant is ionic?

Yes → Use linear equation — 2050

No

Use nonlinear equation — 2055

**FIG. 20**

<u>2100</u>

Receive (i) an indication of a base nonionic surfactant, and/or (ii) a molar fraction of the base nonionic surfactant — 2101

Receive (i) a desired hydrophilic lipophilic difference (HLD) range of a solution, (ii) an optimum temperature of the solution, (iii) an effective alkane carbon number (EACN) of an oil of the solution, and/or (iv) a salinity of the solution — 2105

Retrieve list of surfactants — 2110

Create nonlinear equations — 2115

Determine recommended surfactants — 2120

Rank and display recommended surfactants — 2125

Receive selection of surfactant — 2130

Produce surfactant mix and/or solution including the surfactant mix — 2135

# FIG. 21

**FIG. 22**

EP 4 451 279 A1

FIG. 23

<table>
<tr><td colspan="3">Europäisches Patentamt<br>European Patent Office<br>Office européen des brevets</td><td colspan="2" align="center">**EUROPEAN SEARCH REPORT**</td><td>Application Number<br>**EP 23 16 9108**</td></tr>
</table>

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GHAYOUR A.: "A methodology for measuring the characteristic curvature of technical-grade ethoxylated nonionic surfactants: the effects of concentration and dilution",<br>TENSIDE, SURFACTANTS, DETERGENTS,<br>vol. 60, no. 1,<br>15 December 2022 (2022-12-15), pages 1-12,<br>XP093085668,<br>DE<br>ISSN: 0932-3414, DOI:<br>10.1515/tsd-2022-2464<br>* the whole document *<br>----- | 1-9,<br>15-20 | INV.<br>G16C20/30 |
| X | ZARATE-MUÑOZ S. ET AL: "A Simplified Methodology to Measure the Characteristic Curvature (Cc) of Alkyl Ethoxylate Nonionic Surfactants",<br>JOURNAL SURFACTDETERG,,<br>vol. 19, no. 2,<br>19 January 2016 (2016-01-19), pages 249-263, XP035772130,<br>ISSN: 1097-3958, DOI:<br>10.1007/S11743-016-1787-X<br>[retrieved on 2016-01-19]<br>* the whole document *<br>----- | 1-9,<br>15-20 | |
| | | | TECHNICAL FIELDS SEARCHED (IPC)<br><br>G16C<br>C11D<br>C09K |

-/--

The present search report has been drawn up for all claims

| Place of search | Date of completion of the search | Examiner |
|---|---|---|
| The Hague | 2 February 2024 | Godzina, Przemyslaw |

EPO FORM 1503 03.82 (P04C01)

**page 1 of 3**

Europäisches
Patentamt

European
Patent Office

Office européen
des brevets

**EUROPEAN SEARCH REPORT**

Application Number

EP 23 16 9108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DO L. D. ET AL: "Detergency of Vegetable Oils and Semi-Solid Fats Using Microemulsion Mixtures of Anionic Extended Surfactants: The HLD Concept and Cold Water Applications", JOURNAL OF SURFACTANTS AND DETERGENTS, SPRINGER/OACS, USA, vol. 18, no. 3, 1 May 2015 (2015-05-01), pages 373-382, XP001595278, ISSN: 1558-9293, DOI: 10.1007/S11743-014-1659-1 [retrieved on 2014-12-14] * the whole document * | 1-9, 15-20 | |
| A | HAMMOND C. E. ET AL: "On the Characteristic Curvature of Alkyl-Polypropylene Oxide Sulfate Extended Surfactants", JOURNAL OF SURFACTANTS AND DETERGENTS, SPRINGER/OACS, USA, vol. 15, no. 2, 30 October 2011 (2011-10-30), pages 157-165, XP035015881, ISSN: 1558-9293, DOI: 10.1007/S11743-011-1303-2 * the whole document * | 1-9, 15-20 | |
| X | CHENG K. C. ET AL: "Design and performance optimisation of detergent product containing binary mixture of anionic-nonionic surfactants", HELIYON, vol. 6, no. 5, 1 May 2020 (2020-05-01), page e03861, XP093122456, GB ISSN: 2405-8440, DOI: 10.1016/j.heliyon.2020.e03861 | 10-13 | TECHNICAL FIELDS SEARCHED (IPC) |
| A | * the whole document * | 14 | |

-/--

The present search report has been drawn up for all claims

| Place of search | Date of completion of the search | Examiner |
|---|---|---|
| The Hague | 2 February 2024 | Godzina, Przemyslaw |

EPO FORM 1503 03.82 (P04C01)

**EUROPEAN SEARCH REPORT**

Application Number

EP 23 16 9108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/397035 A1 (JIN LUCHAO [US] ET AL) 15 December 2022 (2022-12-15) * the whole document * ----- | 10-14 | |

TECHNICAL FIELDS SEARCHED (IPC)

The present search report has been drawn up for all claims

| Place of search | Date of completion of the search | Examiner |
|---|---|---|
| The Hague | 2 February 2024 | Godzina, Przemyslaw |

EPO FORM 1503 03.82 (P04C01)

page 3 of 3

Europäisches
Patentamt

European
Patent Office

Office européen
des brevets

**Application Number**

**EP 23 16 9108**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

## LACK OF UNITY OF INVENTION
## SHEET B

**Application Number**

**EP 23 16 9108**

The Search Division considers that the present European patent application does not comply with the requirements of unity of invention and relates to several inventions or groups of inventions, namely:

```
1. claims: 1-9, 15-20

    A computer-implemented method for determining a
    characteristic value of a nonionic surfactant mix.
                        ---


2. claims: 10-14

    A computer-implemented method for determining an optimal
    surfactant.
                        ---
```

**ANNEX TO THE EUROPEAN SEARCH REPORT
ON EUROPEAN PATENT APPLICATION NO.**

EP 23 16 9108

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2024

| Patent document cited in search report | | Publication date | Patent family member(s) | | Publication date |
|---|---|---|---|---|---|
| US 2022397035 | A1 | 15-12-2022 | CA | 3162084 A1 | 14-12-2022 |
| | | | US | 2022397035 A1 | 15-12-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ACOSTA EJ ; BHAKTA AS.** The HLDNAC Model for Mixtures of Ionic and Nonionic Surfactants. *J Surfactants Deterg,* 2009, vol. 12, 7-19
- **WITTHAYAPANYANON A ; HARWELL JH ; SABATINI DA.** Hydrophilic-lipophilic deviation (HLD) method for characterizing conventional and extended surfactants. *J Colloid Interface Sci,* 2008, vol. 325, 259-266
- **SALAGER JL ; MORGAN JC ; SCHECHTER RS ; WADE WH ; VASQUEZ E.** Optimum Formulation of Surfactant/Water/Oil Systems for Minimum Interfacial Tension or Phase Behavior. *Soc Pet Eng J,* 1979, vol. 19 (02), 107-115
- **SALAGER JL ; BOURREL M ; SCHECHTER RS ; WADE WH.** Mixing Rules for Optimim Phase-Behavior Formulations of Surfactant/Oil/Water Systems. *Soc Pet Eng J,* 1979, vol. 19 (05), 271-278